(19) Europäisches Patentamt
European Patent Office
Office européen des brevets

(11) Veröffentlichungsnummer : **0 146 787**
**B1**

(12) # EUROPÄISCHE PATENTSCHRIFT

(45) Veröffentlichungstag der Patentschrift : **17.08.88**

(51) Int. Cl.[4] : **C 07 D403/12**, C 07 D491/056, A 61 K 31/55

(21) Anmeldenummer : **84114061.9**

(22) Anmeldetag : **22.11.84**

(54) **Indolderivate, diese Verbindungen enthaltende Arzneimittel und Verfahren zu ihrer Herstellung.**

(30) Priorität : **03.12.83 DE 3343801**

(43) Veröffentlichungstag der Anmeldung : **03.07.85 Patentblatt 85/27**

(45) Bekanntmachung des Hinweises auf die Patenterteilung : **17.08.88 Patentblatt 88/33**

(84) Benannte Vertragsstaaten : **AT BE CH DE FR GB IT LI LU NL SE**

(56) Entgegenhaltungen :
**EP-A- 0 065 229**
**EP-B- 0 000 151**
**US-A- 4 059 583**
**US-A- 4 462 933**
**Die Akte enthält technische Angaben, die nach dem Eingang der Anmeldung eingereicht wurden und die nicht in dieser Patentschrift enthalten sind.**

(73) Patentinhaber : **Dr. Karl Thomae GmbH**
**Postfach 1755**
**D-7950 Biberach (Riss) (DE)**

(72) Erfinder : **Reiffen, Manfred, Dr. Dipl.-Chem.**
**Matthias-Erzberger-Strasse 40**
**D-7950 Biberach 1 (DE)**
Erfinder : **Heider, Joachim, Dr. Dipl.-Chem.**
**Am Hang 3**
**D-7951 Warthausen 1 (DE)**
Erfinder : **Austel, Volkhard, Dr. Dipl.-Chem.**
**Kapellenweg 7**
**D-7950 Biberach 1 (DE)**
Erfinder : **Hauel, Norbert, Dr. Dipl.-Chem.**
**Händelstrasse 12**
**D-7950 Biberach 1 (DE)**
Erfinder : **Kobinger, Walter, Prof. Dr.**
**Belghofergasse 27**
**A-1121 Wien (AT)**
Erfinder : **Lillie, Christian, Dr.**
**Hansi-Niese-Weg 12**
**A-1130 Wien (AT)**

**EP 0 146 787 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

In der EP-A-0 065 229 werden bereits Verbindungen beschrieben, die wertvolle pharmakologische Eigenschaften aufweisen, insbesondere eine langanhaltende herzfrequenzsenkende Wirkung und eine herabsetzende Wirkung auf den $O_2$-Bedarf des Herzens.

Überraschenderweise wurde nun gefunden, daß die neuen Indolderivate der allgemeinen Formel I

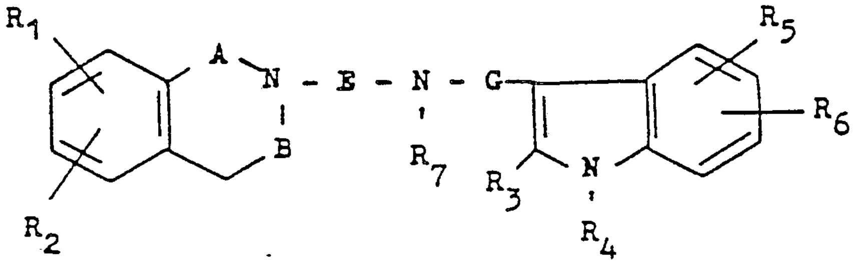

(I)

bei äquivalenten biologischen Eigenschaften eine geringere Nebenwirkung auf den Tremor aufweisen.

Gegenstand dieser Patentschrifs sind somit die neuen Indolderivate der obigen allgemeinen Formel, deren Säureadditionssalze, insbesondere deren physiologisch verträgliche Säureadditionssalze mit anorganischen oder organischen Säuren, Verfahren zu ihrer Herstellung und diese Verbindungen enthaltende Arzneimittel.

In der obigen allgemeinen Formel I bedeutet

A eine $-CH_2-CH_2-$, $-CH=CH-$, $-\underset{x}{N}H-CO-$ oder $-\underset{x}{C}H_2-CO$-Gruppe

und

B eine Methylen-, Carbonyl- oder Thiocarbonylgruppe oder

A eine $-CO-CO-$ oder

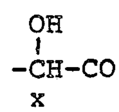

Gruppe und B eine Methylengruppe, wobei das mit x gekennzeichnete Kohlenstoffatom jeweils mit dem Phenylkern verknüpft ist,

E eine gegebenenfalls durch eine Alkylgruppe mit 1 bis 3 Kohlenstoffatomen substituierte geradkettige Alkylengruppe mit 2 bis 4 Kohlenstoffatomen, eine 2-Hydroxy-n-propylen-, 2-Hydroxy-n-butylen- oder 3-Hydroxy-n-butylengruppe,

G eine gegebenenfalls durch eine Alkylgruppe mit 1 bis 3 Kohlenstoffatomen substituierte geradkettige Alkylengruppe mit 1 bis 5 Kohlenstoffatomen,

$R^1$ ein Wasserstoff-, Chlor- oder Bromatom, eine Trifluormethyl-, Nitro-, Amino-, Alkylamino-, Dialkylamino-, Alkyl-, Hydroxy-, Alkoxy- oder Phenylalkoxygruppe, wobei jeder Alkylteil jeweils 1 bis 3 Kohlenstoffatome enthalten kann,

$R^2$ ein Wasserstoff-, Chlor- oder Bromatom, eine Hydroxy-, Alkoxy-, Phenylalkoxy- oder Alkylgruppe, wobei jeder Alkylteil jeweils 1 bis 3 Kohlenstoffatome enthalten kann, oder

$R^1$ und $R^2$ zusammen eine Alkylendioxygruppe mit 1 oder 2 Kohlenstoffatomen,

$R^3$ ein Wasserstoff-, Chlor- oder Bromatom oder eine Alkylgruppe mit 1 bis 4 Kohlenstoffatomen,

$R^4$ ein Wasserstoffatom, eine Alkyl- oder Phenylalkylgruppe, wobei der Alkylteil jeweils 1 bis 3 Kohlenstoffatome enthalten kann,

$R^5$ ein Wasserstoff-, Fluor-, Chlor- oder Bromatom, eine Alkyl-, Hydroxy-, Alkoxy- oder Phenylalkoxygruppe, wobei jeder Alkylteil jeweils 1 bis 3 Kohlenstoffatome enthalten kann,

$R^6$ ein Wasserstoffatom oder eine Alkoxygruppe mit 1 bis 3 Kohlenstoffatomen und

$R^7$ ein Wasserstoffatom, eine Alkenylgruppe mit 3 bis 5 Kohlenstoffatomen, eine Alkyl- oder Phenylalkylgruppe, wobei der Alkylteil jeweils 1 bis 3 Kohlenstoffatomen enthalten kann.

Für die bei der Definition der Reste eingangs erwähnten Bedeutungen kommt beispielsweise

für $R_1$ die des Wasserstoff-, Chlor- oder Bromatoms, der Methyl-, Ethyl-, n-Propyl-, Isopropyl-, Trifluormethyl-, Hydroxy-, Methoxy-, Ethoxy-, n-Propoxy, Isopropoxy-, Nitro-, Amino-, Methylamino-, Ethylamino-, n-Propylamino-, Isopropylamino-, Dimethylamino-, Diethylamino-, Di-,-propylamino-, Diisopropylamino-, Methyl-ethylamino-, Methyl-n-propylamino-, Methyl-isopropylamino-, Ethyl-n-propylamino-, Benzyloxy-, 1-Phenylethoxy-, 1-Phenylpropoxy-, 2-Phenylethoxy- oder 3-Phenylpropoxygruppe,

für $R_2$ die des Wasserstoff-, Chlor- oder Bromatoms, der Methyl-, Ethyl-, n-Propyl-, Isopropyl-, Hydroxy-, Methoxy-, Ethoxy-, n-Propoxy, Isopropoxy-, Benzyloxy-, 1-Phenylethoxy-, 2-Phenylethoxy-, 2-Phenylpropoxy- oder 3-Phenylpropoxygruppe oder zusammen mit $R_1$ die der Methylendioxy- oder Ethylendioxygruppe,

für $R_3$ die des Wasserstoff-, Chlor- oder Bromatoms, der Methyl-, Ethyl-, n-Propyl-, Isopropyl-, n-Butyl-, Isobutyl- oder tert.-Butylgruppe,

für $R_4$ die des Wasserstoffatoms, der Methyl-, Ethyl-, n-Propyl-, Isopropyl-, Benzyl, 1-Phenylethyl-, 2-Phenylethyl- oder 3-Phenylpropylgruppe,

für $R_5$ die des Wasserstoff-, Fluor-, Chlor- oder Bromatoms, der Methyl-, Ethyl-, n-Propyl-, Isopropyl-, Hydroxy-, Methoxy-, Ethoxy-, n-Propoxy-, Isopropoxy-, Benzyloxy-, 1-Phenylethoxy-, 2-Phenylethoxy-, 1-Phenylpropoxy- oder 3-Phenylpropoxygruppe,

für $R_6$ die des Wasserstoffatoms, der Methoxy-, Ethoxy-, n-Propoxy- oder Isopropoxygruppe,

für $R_7$ die des Wasserstoffatoms, der Methyl-, Ethyl-, n-Propyl-, Isopropyl-, Benzyl-, 1-Phenylethyl-, 2-Phenylethyl-, 1-Phenylpropyl-, 1-Methyl-1-phenylethyl-, 3-phenylpropyl, Allyl-, n-Buten-(2)-yl- oder n-Penten-(2)-ylgruppe,

für E die der Ethylen-, n-Propylen-, n-Butylen-, 1-Methylethylen-, 2-Ethyl-ethylen-, 1-Propyl-ethylen-, 1-Methyl-n-propylen-, 2-Methyl-n-propylen-, 1-Ethyl-n-propylen-, 3-Ethyl-n-propylen-, 2-Propyl-n-propylen-, 2-Methyl-n-butylen-, 2-Hydroxy-n-propylen-, 2-Hydroxy-n-butylen- oder 3-Hydroxy-n-butylengruppe und

für G die der Methylen-, Ethyliden-, n-Propyliden-, n-Butyliden-, 2-Methyl-propyliden-, Ethylen, 1-Methyl-ethylen-, 2-Ethyl-ethylen-, 1-Propyl-ethylen-, 2-Methyl-ethylen-, n-Propylen-, n-Butylen-, n-Pentylen-, 1-Methyl-n-propylen-, 1-Methyl-n-butylen-, 1-Methyl-n-pentylen-, 1-Ethyl-n-propylen-, 2-Ethyl-n-propylen- oder 1-Ethyl-n-butylengruppe in Betracht, hierbei steht der Rest $R_1$ bevorzugt in 7-Stellung und $R_2$ in 8-Stellung des Phenylkerns.

Bevorzugte Verbindungen sind jedoch die Verbindungen der allgemeinen Formel Ia

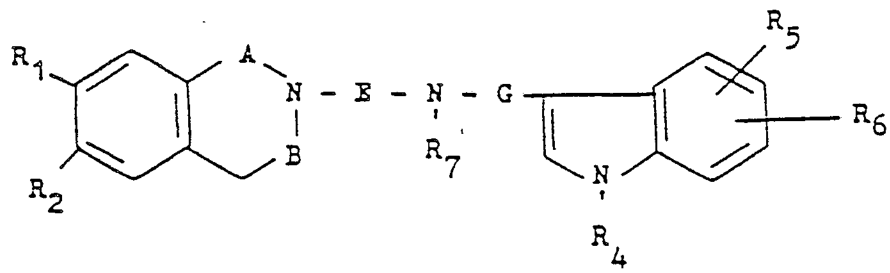

(Ia)

in der

A und B wie eingangs definiert sind,

E eine n-Propylengruppe,

G eine Ethylen- oder n-Propylengruppe,

$R_1$ ein Chloratom- oder Bromatom, eine Methyl-, Methoxy-, Nitro-, Amino-, Methylamino- oder Dimethylaminogruppe,

$R_2$ ein Wasserstoff-,Chlor- oder Bromatom, eine Methoxygruppe oder $R_1$ und $R_2$ zusammen eine Methylendioxygruppe,

$R_4$ ein Wasserstoffatom oder eine Methylgruppe,

$R_5$ ein Wasserstoff-, Fluor-, Chlor- oder Bromatom, eine Methyl-, Hydroxy-, Methoxy- oder Benzyloxygruppe,

$R_6$ ein Wasserstoffatom oder eine Methoxygruppe und

$R_7$ ein Wasserstoffatom oder eine Methylgruppe bedeuten, und deren Säureadditionssalze, insbesondere deren physiologisch verträgliche Säureadditionssalze mit anorganischen oder organischen Säuren.

Besonders bevorzugte Verbindungen der obigen allgemeinen Formel Ia sind jedoch diejenigen, in denen

A eine $-CH_2-CH_2-$ oder $-CH=CH$-Gruppe und B eine Carbonylgruppe oder

A eine $-COCO-$ oder $-NH-CO$-Gruppe und B eine Methylengruppe,

E eine n-Propylengruppe,

G eine Ethylengruppe,

$R_1$ und $R_2$ je eine Methoxygruppe,

$R_4$, $R_5$ und $R_6$ je ein Wasserstoffatom und $R_7$ eine Methylgruppe bedeuten, und deren Säureadditionssalze, insbesondere deren physiologisch verträgliche Säureadditionssalze mit anorganischen oder organischen Säuren.

Erfindungsgemäß erhält man die neuen Verbindungen nach folgenden Verfahren :

a) Umsetzung einer Verbindung der allgemeinen Formel II

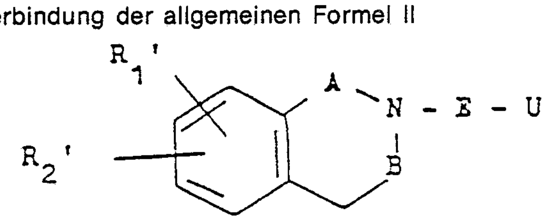

(II)

mit einer Verbindung der allgemeinen Formel III

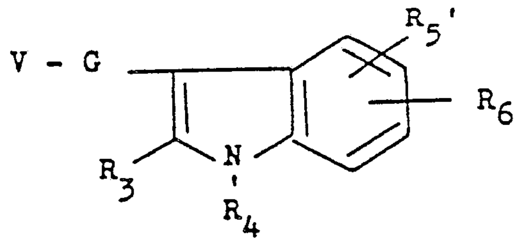

(III)

in der

$R_3$, $R_4$, $R_6$, A, B, E und G wie eingangs definiert sind,

$R_1'$ eine durch einen Schutzrest geschützte Hydroxy-, Amino- oder Alkylaminogruppe darstellt oder die für $R_1$ eingangs erwähnten Bedeutungen besitzt,

$R_2'$ eine durch einen Schutzrest geschützte Hydroxygruppe darstellt oder die für $R_2$ eingangs erwähnten Bedeutungen besitzt,

$R_5'$ eine durch einen Schutzrest geschützte Hydroxygruppe darstellt oder die für $R_5$ eingangs erwähnten Bedeutungen besitzt, einer der Reste U oder V die $R_7'$ —NH-Gruppe, wobei $R_7'$ eine Schutzgruppe für eine Aminogruppe darstellt oder die

für $R_7$ eingangs erwähnten Bedeutungen besitzt, und der andere der Reste U oder V eine nukleophile Austrittsgruppe wie ein Halogenatom oder eine Sulfonyloxygruppe, z. B. ein Chlor-, Brom-, oder Jodatom, die Methansulfonyloxy-, p-Toluolsulfonyloxy- oder Ethoxysulfonyloxygruppe, darstellt und gegebenenfalls anschließende Abspaltung eines verwendeten Schutzrestes.

Als Schutzrest für eine Hydroxygruppe kommt beispielsweise die Trimethylsilyl-, Acetyl-, Benzoyl-, Benzyl- oder Tetrahydropyranylgruppe und als Schutzrest für eine Amino- oder Alkylaminogruppe die Acetyl-, Benzoyl-, Ethoxycarbonyl- oder Benzylgruppe in Betracht.

Die Umsetzung wird zweckmäßigerweise in einem Lösungsmittel oder Lösungsmittelgemisch wie Aceton, Diethylether, Methylformamid, Dimethylformamid, Dimethylsulfoxid, Benzol, Chlorbenzol, Tetrahydrofuran, Benzol/Tetrahydrofuran, Dioxan oder in einem Überschuß der eingesetzten Verbindungen der allgemeinen Formeln II und/oder III und gegebenenfalls in Gegenwart eines säurebindenden Mittels, z. B. eines Alkoholats wie Kalium-tert.-butylat, eines Alkalihydroxids wie Natrium- oder Kaliumhydroxid, eines Alkalicarbonats wie Kaliumcarbonat, eines Alkaliamids wie Natriumamid, eines Alkalihydrids wie Natriumhydrid, einer tertiären organischen Base wie Triethylamin oder Pyridin, wobei die letzteren gleichzeitig auch als Lösungsmittel dienen können, oder eines Reaktionsbeschleunigers wie Kaliumjodid je nach der Reaktionsfähigkeit des nukleophil austauschbaren Restes zweckmäßigerweise bei Temperaturen zwischen 0 und 150 °C, vorzugsweise bei Temperaturen zwischen 50 und 120 °C, z. B. bei der Siedetemperatur des verwendeten Lösungsmittels, durchgeführt. Die Umsetzung kann jedoch auch ohne Lösungsmittel durchgeführt werden. Besonders vorteilhaft wird die Umsetzung jedoch in Gegenwart einer tertiären organischen Base oder eines Überschusses des eingesetzten Amins der allgemeinen Formel III durchgeführt.

Die gegebenenfalls anschließende Abspaltung eines verwendeten Schutzrestes erfolgt vorzugsweise hydrolytisch in einem wässrigen Lösungsmittel, z. B. in Wasser, Isopropanol/Wasser, Tetrahydrofuran/Wasser oder Dioxan/Wasser, in Gegenwart einer Säure wie Salzsäure oder Schwefelsäure oder in Gegenwart einer Alkalibase wie Natriumhydroxid oder Kaliumhydroxid bei Temperaturen zwischen 0 und 100 °C, vorzugsweise bei der Siedetemperatur des Reaktionsgemisches. Die Abspaltung eines Benzylrestes erfolgt jedoch vorzugsweise hydrogenolytisch, z. B. mit Wasserstoff in Gegenwart eines Katalysators wie Palladium/Kohle in einem Lösungsmittel wie Methanol, Ethanol, Essigsäureethylester oder Eisessig gegebenenfalls unter Zusatz einer Säure wie Salzsäure bei Temperaturen zwischen 0 und 50 °C, vorzugsweise jedoch bei Raumtemperatur, und einem Wasserstoffdruck von 1 bis 7 bar, vorzugsweise jedoch von 3 bis 5 bar.

b) Zur Herstellung von Verbindungen der allgemeinen Formel I, in der B eine Thiocarbonylgruppe darstellt:

Umsetzung einer Verbindung der allgemeinen Formel IV

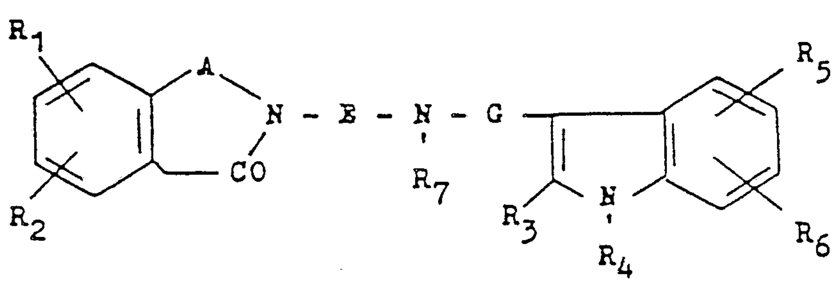

(IV)

in der $R_1$ bis $R_7$, A, E und G wie eingangs definiert sind, mit einem schwefeleinführenden Mittel.

Die Umsetzung wird mit einem schwefeleinführenden Mittel wie Phosphorpentasulfid oder 2,4-Bis-(4-methoxyphenyl)-1,3-dithia-2,4-diphosphetan-2,4-disulfid zweckmäßigerweise in einem Lösungsmittel wie Toluol oder Xylol bei Temperaturen zwischen 50 und 150 °C, z. B. bei der Siedetemperatur des Reaktionsgemisches, durchgeführt.

c) Zur Herstellung von Verbindungen der allgemeinen Formel I, in der A eine

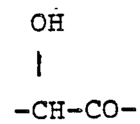

Gruppe darstellt : Reduktion einer Verbindung der allgemeinen Formel V

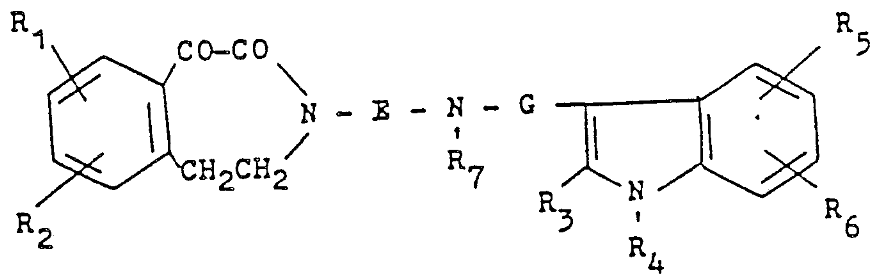

(V)

in der $R_1$ bis $R_7$, E und G wie eingangs definiert sind.

Die Umsetzung wird in Gegenwart eines geeigneten Reduktionsmittels wie einem Metallhydrid, z. B. Natriumborhydrid, in einem geeigneten Lösungsmittel wie Wasser/Methanol oder Methanol/Ether, bei Temperaturen zwischen 0 und 80 °C, vorzugsweise jedoch bei Temperaturen zwischen 15 und 40 °C, durchgeführt.

d) Zur Herstellung von Verbindungen der allgemeinen Formel I, in der A eine $-CH_2CH_2-$ oder $-CH=CH-$ und B eine Methylengruppe darstellen :

Reduktion einer Verbindung der allgemeinen Formel VI

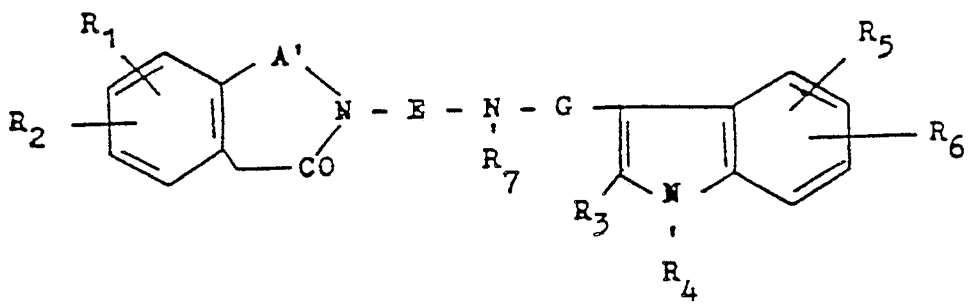

(VI)

in der

$R_1$ bis $R_7$, E und G wie eingangs definiert sind und

A' eine $-CH_2-CH_2-$ oder $-CH=CH$-Gruppe darstellt.

Die Reduktion wird vorzugsweise mit einem Metallhydrid wie Lithiumaluminiumhydrid oder Diboran oder mit einem Komplex aus Boran und einem Thioäther, z. B. mit Boran-Dimethylsulfid-Komplex, in einem geeigneten Lösungsmittel wie Diethylether oder Tetrahydrofuran bei Temperaturen zwischen 0 und 50 °C, vorzugsweise jedoch bei temperaturen zwischen 10 und 25 °C, durchgeführt.

e) Zur Herstellung von Verbindungen der allgemeinen Formel I, in der A die $-COCO$-Gruppe darstellt : Oxidation einer Verbindung der allgemeinen Formel VII

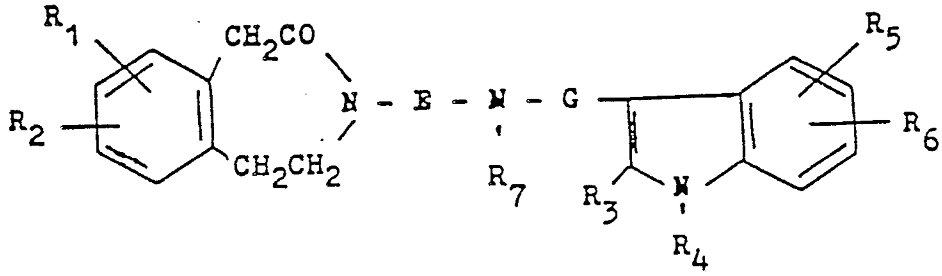

(VII)

in der $R_1$ bis $R_7$, E und G wie eingangs definiert sind.

Die Oxidation wird vorzugsweise mit einem Oxidationsmittel wie Kaliumpermanganat, Selendioxid oder Natriumdichromat in einem geeigneten Lösungsmittel oder Lösungsmittelgemisch wie Wasser, Wasser/Dioxan, Eisessig, Wasser/Essigsäure oder Acetanhydrid bei Temperaturen zwischen 0 und 100 °C, vorzugsweise bei Temperaturen zwischen 20 und 80 °C, durchgeführt.

f) Zur Herstellung von Verbindungen der allgemeinen Formel I, in der A die —NH—CO-Gruppe und E eine gegebenenfalls durch eine Alkylgruppe mit 1 bis 3 Kohlenstoffatomen substituierte Alkylengruppe mit 2 bis 4 Kohlenstoffatomen darstellen :

Umsetzung einer Verbindung der allgemeinen Formel VIII

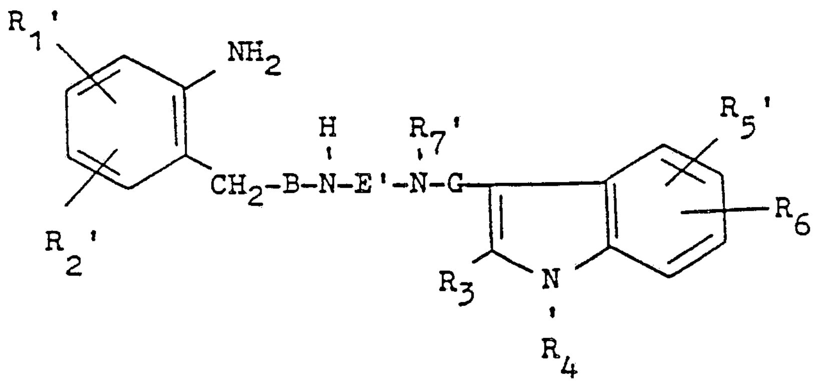

(VIII)

in der

B, G, $R_3$, $R_4$ und $R_6$ wie eingangs definiert sind,

E' eine gegebenenfalls durch eine Alkylgruppe mit 1 bis 3 Kohlenstoffatomen substituierte Alkenylgruppe mit 2 bis 4 Kohlenstoffatomen,

$R_1'$ eine durch eine Schutzgruppe geschützte Hydroxy-, Amino-, oder Alkylaminogruppe darstellt oder die für $R_1$ eingangs erwähnten Bedeutungen besitzt,

$R_2'$ eine durch eine Schutzgruppe geschützte Hydroxygruppe darstellt oder die für $R_2$ eingangs erwähnten Bedeutungen besitzt,

$R_5'$ eine durch eine Schutzgruppe geschützte Hydroxygruppe darstellt oder die für $R_5$ eingangs erwähnten Bedeutungen besitzt,

$R_7'$ eine Schutzgruppe für eine Aminogruppe darstellt oder mit Ausnahme von Wasserstoff die für $R_7$ eingangs erwähnten Bedeutungen besitzt, mit einem Kohlensäurederivat der allgemeinen Formel IX

$$W—CO—W \qquad (IX)$$

in der W, die gleich oder verschieden sein können, jeweils eine nukleophile Austrittsgruppe, z. B. ein Chlor- oder Bromatom, eine Alkoxygruppe mit 1 bis 3 Kohlenstoffatomen, eine Imidazolyl-(1)-gruppe oder auch eine Trichlormethoxygruppe, wenn der andere der Reste W ein Chlor- oder Bromatom darstellt, bedeuten, und gegebenenfalls anschließende Abspaltung eines verwendeten Schutzrestes.

Als Schutzrest für eine Hydroxygruppe kommt beispielsweise die Trimethylsilyl-, Acetyl-, Benzoyl-, Benzyl- oder Tetrahydropyranylgruppe und als Schutzrest für eine Amino- oder Alkylaminogruppe die Acetyl-, Benzoyl-, Ethoxycarbonyl- oder Benzylgruppe in Betracht.

Die Umsetzung wird zweckmäßigerweise in einem Lösungsmittel oder Lösungsmittelgemisch wie Methylenchlorid, Tetrachlorkohlenstoff, Benzol, Tetrahydrofuran, Benzol/Tetrahydrofuran, Dioxan oder Acetonitril zweckmäßigerweise bei Temperaturen zwischen 0 und 150 °C, vorzugsweise jedoch bei der Siedetemperatur des verwendeten Lösungsmittels, z. B. bei Temperaturen zwischen 40 und 100 °C, und gegebenenfalls in Gegenwart eines säurebindenden Mittels wie Kaliumcarbonat, Natriumhydroxid, Kaliumhydroxid, Pyridin oder Triethylamin, wobei die letzteren gleichzeitig auch als Lösungsmittel dienen können, durchgeführt. Die Umsetzung kann jedoch auch ohne Lösungsmittel durchgeführt werden. Bedeutet in einer eingesetzten Verbindung der allgemeinen Formel IV mindestens einer der Reste W eine Alkoxygruppe mit 1 bis 3 Kohlenstoffatomen, so wird die Umsetzung vorzugsweise in einem Überschuß des eingesetzten Esters als Lösungsmittel durchgeführt.

Die gegebenenfalls anschließende Abspaltung eines verwendeten Schutzrestes erfolgt vorzugsweise hydrolytisch in einem wässrigen Lösungsmittel, z. B. in Wasser, Isopropanol/Wasser, Tetrahydrofuran/Wasser oder Dioxan/Wasser, in Gegenwart einer Säure wie Salzsäure oder Schwefelsäure oder in Gegenwart einer Alkalibase wie Natriumhydroxid oder Kaliumhydroxid bei Temperaturen zwischen 0 und 100 °C, vorzugsweise bei der Siedetemperatur des Reaktionsgemisches. Die Abspaltung eines Benzylrestes kann jedoch auch hydrogenolytisch erfolgen, z. B. mit Wasserstoff in Gegenwart eines Katalysators wie Palladium/Kohle, in einem Lösungsmittel wie Methanol, Ethanol, Essisäureethylester oder Eisessig gegebenenfalls unter Zusatz einer Säure wie Salzsäure bei Temperaturen zwischen 0 und 50 °C, vorzugsweise jedoch bei Raumtemperatur, und einem Wasserstoffdruck von 1 bis 7 bar, vorzugsweise jedoch von 3 bis 5 bar.

Die so erhaltenen Verbindungen der allgemeinen Formel I lassen sich ferner in ihre Säureadditionssalze, insbesondere in ihre physiologisch verträgliche Säureadditionssalze mit anorganischen oder organischen Säuren Überführen. Als Säuren kommen hierbei beispielsweise Salzsäure, Bromwasserstoffsäure, Schwefelsäure, Phosphorsäure, Essigsäure, Milchsäure, Citronensäure, Weinsäure, Bernsteinsäure, Maleinsäure oder Fumarsäure in Betracht.

Die als Ausgansstoffe verwendeten Verbindungen der allgemeinen Formeln II bis IX sind teilweise literaturbekannt bzw. man erhält sie nach an sich bekannten Verfahren.

So erhält man beispielsweise eine Ausgangsverbindung der allgemeinen Formel II durch Umsetzung eines entsprechenden Benzazepins mit einer entsprechenden Halogenverbindung und gegebenenfalls durch anschließende Umsetzung mit einem entsprechenden Amin. Das hierfür erforderliche in 3-Stellung unsubstituierte entsprechende Benzazepin erhält man durch Cyclisierung einer entsprechenden Verbindung, z. B. durch Cyclisierung einer Verbindung der allgemeinen Formel X

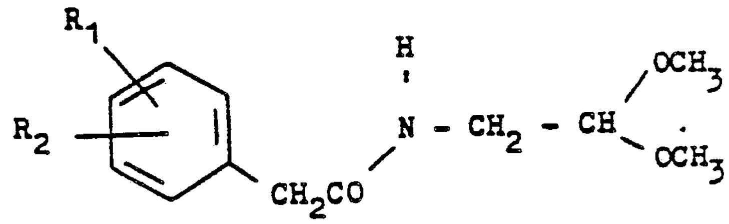

(X)

oder auch der allgemeinen Formel XI

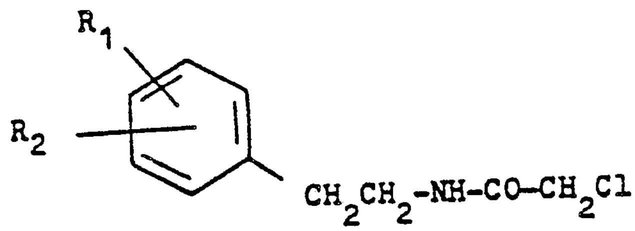

(XI)

gegebenenfalls anschließender katalytischer Hydrierung und/oder Reduktion der Carbonylgruppe beispielsweise mit Natriumborhydrid/Eisessig (siehe EP-A 0 007 070) und/oder Oxidation, z. B. mit Selendioxid.

Eine als Ausgangsstoff verwendete Verbindung der allgemeinen Formeln IV bis VII erhält man vorzugsweise durch Umsetzung einer entsprechenden Halogenverbindung mit einem entsprechenden Amin und gegebenenfalls anschließende Abspaltung von Schutzresten, die zum Schutz von Hydroxy- und/oder Aminogruppen verwendet werden.

Eine als Ausgangsstoff verwendete Verbindung der allgemeinen Formel VIII erhält man beispielsweise durch Reduktion einer entsprechenden Nitroverbindung.

Wie bereits eingangs erwähnt, weisen die neuen Verbindungen der allgemeinen Formel I und deren physiologisch verträgliche Säureadditionssalze mit anorganischen oder organischen Säuren wertvolle pharmakologische Eigenschaften auf, insbesondere bei geringen Nebenwirkungen, z. B. einer geringen antimuskarinischen, eine lang anhaltende herzfrequenzsenkende Wirkung sowie eine Herabsetzung des $O_2$-Bedarfs des Herzens, außerdem weisen sie eine α-blockierende Wirkung auf.

Beispielsweise wurden die Verbindungen

A = 1-(7,8-Dimethoxy-1,3,4,5-tetrahydro-2H-3-benzazepin-2-on-3-yl)-3-[N-methyl-N-(2-(indolyl-3)-ethyl)-amino]-propan

und

B = 1-(7,8-Dimethoxy-1,3,4,5-tetrahydro-2H-3-benzazepin-1,2-dior-3-yl)-3-[N-methyl-N-(2-(indolyl-3)-ethyl)-amino]-propan

auf ihre biologischen Eigenschaften wie folgt untersucht :

Wirkung auf die Herzfrequenz an Ratten :

Die Wirkung der zu untersuchenden Substanzen auf die Herzfrequenz wurde pro Dosis an 2 Ratten mit einem durchschnittlichen Gewicht von 250-300 g untersucht. Hierzu wurden die Ratten mit Pentobarbital (50 mg/kg i. p. und 20 mg/kg s. c.) narkotisiert. Die zu untersuchenden Substanzen wurden in Wäßriger Lösung in die Vena jugularis injiziert (0,1 ml/100 g).

Der Blutdruck wurde über in eine A. carotis eingebundene Kanüle gemessen und die Herzfrequenz wurde aus einem mit Nadelelektroden abgeleiteten EKG (II. oder III. Ableitung) registriert. Die Herzfre-

quenz der Tiere in der Kontrollperiode lagen zwischen 350 und 400 Schlägen/Minuten (S/min).

Die nachfolgende Tabelle enthält die gefundenen Werte :

| Substanz | Dosis [mg/kg] | Herzfrequenzsenkung, gemessen 20 Minuten nach Substanzapplikation [S/min] |
|---|---|---|
| A | 5,0 | - 153 |
| | 2,5 | - 143 |
| | 1,0 | - 99 |
| B | 5,0 | - 128 |

Die erfindungsgemäß hergestellten Verbindungen weisen in therapeutischen Dosen keinerlei toxische Nebenwirkungen auf. So konnten beipielsweise bei einer intravenösen Applikation der Substanz A auch in einer hohen Dosis von 20 mg/kg an Mäusen keine toxischen Nebenwirkungen beobachtet werden.

Aufgrund ihrer pharmakologischen Eigenschaften eignen sich die erfindungsgemäß hergestellen Verbindungen zur Behandlung von Sinustachykardien verschiedener Genese und zur Prophylaxe und Therapie ischämischer Herzerkrankungen.

Die zur Erzielung einer entsprechenden Wirkung erforderliche Dosierung beträgt zweckmäßigerweise ein- bis zweimal täglich 0,03 bis 0,4 mg/kg Körpergewicht, vorzugsweise 0,07 bis 0,25 mg/kg Körpergewicht. Hierzu lassen sich die erfindungsgemäß hergestellten Verbindungen der allgemeinen Formel I sowie ihre physiologisch verträglichen Säureadditionssalze mit anorganischen oder organischen Säuren, gegebenenfalls in Kombination mit anderen Wirksubstanzen, zusammen mit einem oder mehreren inerten üblichen Trägerstoffen und/oder Verdünnungsmitteln, z. B. mit Maisstärke, Milchzucker, Rohrzucker, mikrokristalliner Cellulose, Magnesiumstearat, Polyvinylpyrrolidon, Citronensäure, Weinsäure, Wasser, Wasser/Äthanol, Wasser/Glycerin, Wasser/Sorbit, Wasser/Polyäthylenglykol, Propylenglykol, Carboxymethylcellulose oder fetthaltige Substanzen wie Hartfett oder deren geeignete Gemische, in übliche galenische Zubereitungen wie Tabletten, Dragees, Kapseln, Pulver, Suspensionen, Tropfen, Ampullen, Säfte oder Zäpfchen einarbeiten.

Die nachfolgenden Beispiele sollen die Erfindung näher erläutern :

Herstellung der Ausgangsverbindungen :

### Beispiel A

7,8-Dimethoxy-1,3-dihydro-2H-3-benzazepin-2-on

a) 3,4-Dimethoxy-phenylessigsäurechlorid

Zu einer Suspension von 549,4 g 3,4-Dimethoxy-phenylessigsäure in 600 ml Methylenchlorid werden während 2 Stunden 600 ml Thionylchlorid unter Rühren zugetropft. Nach beendeter Gasentwicklung (16 Stunden) wird noch eine Stunde am Rückfluß gekocht. Nach dem Entfernen der leicht flüchtigen Komponenten wird der Rückstand im Vakuum destilliert.

Ausbeute : 486 g (80,8 % der Theorie),
Kp : 134-136 °C/1,95 mbar

b) N-(2,2-Dimethoxyethyl)-3,4-dimethoxy-phenylacetamid

Unter Eiskühlung wird eine Lösung von 485,2 g 3,4-Dimethoxyphenylessigsäurechlorid in 1,1 l Methylenchlorid bei 15-20 °C zu einer Lösung von 246,2 ml Aminoacetaldehyddimethylacetal und 315 ml Triethylamin in 2,2 l Methylenchlorid zugetropft und eine Stunde bei 16-18 °C nachgerührt. Anschließend wird mehrfach mit Wasser extrahiert, über Magnesiumsulfat getrocknet und eingedampft. Das so erhaltene Öl kirstallisiert langsam durch.

Ausbeute : 608 g (95 % der Theorie),
Schmelzpunkt : 66-69 °C.

c) 7,8-Dimethoxy-1,3-dihydro-2H-3-benzazepin-2-on

Eine Lösung von 600,6 g N-(2,2-Dimethoxyethyl)-3,4-dimethoxy-phenylacetamid in 3 l konzentrierter Salzsäure wird mit 3 l Eisessig versetzt. Nach 17-stündigem Stehenlassen bei Raumtemperatur wird der Ansatz auf Eis gegossen. Die ausgefallenen Kristalle werden abgesaugt, mit Wasser neutral gewaschen und getrocknet.

Ausbeute : 350 g (75,4 % der Theorie),
Schmelzpunkt : 234-237 °C.

Beispiel B

7,8-Dimethyoxy-1,3,4,5-tetrahydro-2H-3-benzazepin-2-on

Eine Suspension von 21,9 g (0,1 Mol) 7,8-Dimethoxy-1,3-dihydro-2H-3-benzazepin-2-on und 1,5 g Palladium/Kohle (10 %ig) in 200 ml Eisessig wird bei 50 °C und einem Wasserstoffdruck von 5 bar hydriert. Nach Abfiltrieren des Katalysators wird das Lösungsmittel in Vakuum eingedampft und der Rückstand in Methylenchlorid aufgenommen. Nach Extraktion mit Natriumbicarbonat-Lösung und Waschen mit Wasser wird über Magnesiumsulfat getrocknet, eingeengt und über Kieselgel mit Methylenchlorid und anschließend mit steigenden Anteilen von Methanol (bis 10 %) gereinigt.
Ausbeute : 12,6 g (57 % der Theorie),
Schmelzpunkt : 188-191 °C.

Beispiel C

7,8-Dimethoxy-2,3,4,5-tetrahydro-1H-3-benzazepin

Zu einer Suspension von 1,3 g (6 mMol) 7,8-Dimethoxy-1,3,4,5-tetrahydro-2H-3-benzazepin-2-on und 1,1 g (3 mMol) Natriumborhydrid in 20 ml Dioxan wird eine Lösung von 1,8 g Eisessig in 10 ml Dioxan getropft, 3 Stunden unter Rückfluß gekocht, eingeengt und mit Wasser zersetzt. Das Gemisch wird 2 mal mit Methylenchlorid ausgeschüttelt, der Extrakt eingeengt und der Rückstand in Ether aufgenommen. Nach Filtration wird der Ether im Vakuum entfernt.
Ausbeute : 1,1 g (92,7 % der Theorie),
Schmelzpunkt : 86-89 °C.

Beispiel D

6,9-Dimethoxy-1,3-dihydro-2H-3-benzazepin-2-on

2,0 g (0,007 Mol) N-(2,2-Dimethoxyethyl)-2,5-dimethoxyphenyl-acetamid werden mit 3 ml Polyphosphorsäure übergossen und 60 Minuten bei 90 °C gerührt. Anschließend wird mit Eiswasser versetzt, das ausgefallene Produkt abgesaugt und getrocknet.
Ausbeute : 0,98 g (64 % der Theorie),
Schmelzpunkt : 188-191 °C.

Beispiel E

7,8-Dimethyl-1,3-dihydro-2H-3-benzazepin-2-on

Hergestellt analog Beispiel D aus N-(2,2-Dimethoxyethyl)-3,4-dimethyl-phenylacetamid und Polyphosphorsäure.
Ausbeute : 40,1 % der Theorie,
Schmelzpunkt : 220-224 °C.

Beispiel F

7,8-Dimethoxy-1,3,4,5-tetrahydro-2H-3-benzazepin-2,4-dion

a) 7,8-Dimethoxy-2-amino-4-brom-1H-3-benzazepin-hydrobromid

3,7 g (0,017 Mol) 3,4-Dimethoxy-o-phenylen-diacetonitril werden in 10 ml Eisessig suspendiert und bei 20 °C mit 12 ml 30 %iger Bromwasserstoffsäure in Eisessig versetzt. 3 Stunden wird bei Raumtemperatur nachgerührt, der ausgefallene Niederschag abgesaugt, mit Eisessig und anschließend mit Aceton/Ether gewaschen und getrocknet.
Ausbeute : 5,3 g (82,8 % der Theorie),
Schmelzpunkt : 210-211 °C (Zers.).

b) 7,8-Dimethoxy-1,3,4,5-tetrahydro-2H-3-benzazepin-2,4-dion

5,3 g (0,014 Mol) 7,8-Dimethoxy-2-amino-4-brom-1H-3-benzazepin-hydrobromid werden in 100 ml 85 °C heißem Wasser gelöst, mit 1,3 g wasserfreiem Natriumacetat versetzt und eine Stunde auf 90 °C erhitzt. Das Reaktionsgemisch wird abgekühlt, abgesaugt, mit kaltem Wasser nachgewaschen und getrocknet.

Ausbeute : 2,9 g (88 % der Theorie),
Schmelzpunkt : 235 °C (Zers.).

Beispiel G

N-[3-(7,8-Dimethoxy-1,3,4,5-tetrahydro-2H-3-benzazepin-2-on-3-yl)-propyl]-methylamin-hydrochlorid

Hergestellt analog Beispiel B durch katalytische Hydrierung von 1-(7,8-Dimethoxy-1,3-dihydro-2H-3-benzazepin-2-on-3-yl)-3-(N-benzyl-methylamino)-propan.
Ausbeute : 87 % der Theorie,
Schmelzpunkt : 110 °C (Zers.).

Beispiel H

7,8-Dimethoxy-2,3-dihydro-1H-3-benzazepin

Eine siedende Suspension von 0,8 g Lithiumaluminiumhydrid in 100 ml absolutem Dioxan wird mit 2,2 g (0,01 Mol) 7,8-Dimethoxy-1,3-dihydro-2H-3-benzazepin-2-on versetzt und anschließend 3 Stunden unter Rückfluß erhitzt. Unter Eiswasserkühlung wird mit 10 %iger Ammoniumchlorid-Lösung versetzt und der gebildete Niederschlag abgesaugt. Das Filtrat wird im Vakuum auf ein Volumen von etwa 20 ml eingeengt, der ausgefallene weiße Niederschlag abgesaugt und mit wenig Dioxan nachgewaschen.
Ausbeute : 0,9 g (43,8 % der Theorie),
Schmelzpunkt : 162-163 °C.

Beispiel I

1-(7,8-Dimethoxy-1,3,4,5-tetrahydro-2H-3-benzazepin-2-on-3-yl)-3-chlor-propan

a) 1-(7,8-Dimethoxy-1,3-dihydro-2H-3-benzazepin-2-on-3-yl)-3-chlor-propan

131,5 g (0,6 Mol) 7,8-Dimethoxy-1,3-dihydro-2H-3-benzazepin-2-on werden in 900 ml Dimethylsulfoxid suspendiert und unter Rühren mit 80,8 g (0,72 Mol) Kalium-tert. butylat versetzt. Nach 10 Minuten wird die erhaltene Lösung unter Kühlung mit Eiswasser zu 77 ml (0,72 Mol) 1-Brom-3-chlorpropan in 300 ml Dimethylsulfoxid getropft. Nach einer Stunde gießt man auf Eiswasser. Nach kurzer Zeit beginnt die schmierige Fällung zu kristallisieren. Der Niederschlag wird abgesaugt, in Aceton gelöst, mit Wasser nochmals ausgefällt, abgesaugt und getrocknet.
Ausbeute : 155,5 g (87,3 % der Theorie),
Schmelzpunkt : 101-103 °C.

b) 1-(7,8-Dimethoxy-1,3,4,5-tetrahydro-2H-3-benzazepin-2-on-yl)-3-chlor-propan

59,2 g (0,2 Mol) 1-(7,8-Dimethoxy-1,3-dihydro-2H-3-benzazepin-2-on-3-yl)-3-chlor-propan werden in 500 ml Eisessig in Anwesenheit von 5 g 10 %iger Palladium-Kohle 6 Stunden bei 50 °C und 5 bar hydriert. Der Katalysator wird abgesaugt, der Eisessig im Vakuum abdestilliert und der Rückstand nach Zugabe von Wasser mit Kaliumcarbonat neutralisiert. Der Niederschlag wird abgesaugt, mit Wasser salzfrei gewaschen und getrocknet.
Ausbeute : 53 g (89 % der Theorie),
Schmelzpunkt : 85-86 °C.

Beispiel J

3-(N-Methyl-2-amino-ethyl)-indol

a) 3-(N-Formyl-2-amino-ethyl)-indol

20,5 g (0,128 Mol) Tryptamin werden als Suspension in 400 ml Toluol mit 5,3 ml (0,14 Mol) Ameisensäure versetzt. Nach 14-stündigem Kochen am Wasserabscheider, wobei nach je 2 Stunden je 5,3 ml (0,14 Mol) Ameisensäure zugegeben werden, wird einrotiert, in Methylenchlorid gelöst, mit Wasser extrahiert, über Magnesiumsulfat getrocknet, erneut einrotiert und über 2 000 g Aluminiumoxid neutral Aktivität II mit Methylenchlorid und anschließend mit steigenden Anteilen von Ethanol (bis 100 %) gereinigt.
Ausbeute : 23,8 g (98,8 % der Theorie),
IR-Spektrum (Methylenchlorid) : 1 695 $cm^{-1}$ C = 0

b) 3-(N-Methyl-2-amino-ethyl)-indol

Eine Suspension von 6,8 g (0,18 Mol) Lithiumaluminiumhydrid in 150 ml absolutem Tetrahydrofuran wird mit 23,6 g (0,125 Mol) 3-(N-Formyl-2-amino-ethyl)-indol versetzt und anschließend 12 Stunden unter Rückfluß erhitzt. Unter Eiswasserkühlung wird mit Wasser und 15 %iger Natronlauge zersetzt und der gebildete Niederschlag abgesaugt. Das Filtrat wird im Vakuum eingeengt und über 2 000 g Aluminiumoxid neutral Aktivität II mit Methylenchlorid und steigenden Anteilen von Ethanol (bis 20 %) gereinigt.

Ausbeute : 19,6 g (89,9 % der Theorie),
IR-Spektrum (Methylenchlorid) : 3 490 $cm^{-1}$ (Indol)

Beispiel K

5-Methoxy-3-(N-methyl-2-amino-ethyl)-indol

a) 5-Methoxy-indolyl-3-(N-methyl-glyoxamid)

Eine Lösung von 7,9 g (0,054 Mol) 5-Methoxy-indol in 160 ml absolutem Ether wird unter Eiskühlung mit 4,3 ml (0,06 Mol) Oxalylchlorid versetzt. Der erhaltene Niederschlag wird abgesaugt, kurz getrocknet und unter Eiskühlung in 100 ml 40 %ige wässrige Methylamin-Lösung eingetragen, nach 30 Minuten abgesaugt, mit Wasser gewaschen und getrocknet.

Ausbeute : 9,55 g (84,3 % der Theorie),
Schmelzpunkt : 206-207 °C.

b) 5-Methoxy-3-(N-methyl-2-amino-ethyl)-indol

Eine Lösung von 4,64 g (0,02 Mol) 5-Methoxy-indolyl-3-(N-methyl-glyoxamid) und 12,3 ml (0,1 Mol) Bortrifluorid x Diethylether-Komplex in 1 000 ml absolutem Tetrahydrofuran wird unter Stickstoff-Begasung mit 60 ml (0,12 Mol) einer 2 molaren Lösung von Boran-Dimethylsulfid-Komplex versetzt. Nach 70-stündigem Rühren bei Raumtemperatur wird eingeengt im Vakuum und über 900 g Aluminiumoxid neutral Aktivität II mit Methylenchlorid und anschließend steigenden Anteilen von Ethanol (bis 50 %) gereinigt.

Ausbeute : 2,62 g (64 % der Theorie),
Schmelzpunkt : 101-102 °C.

Beispiel L

1-(7-Brom-8-methoxy-1,3,4,5-tetrahydro-2H-3-benzazepin-2-on-3-yl)-3-chlor-propan

a) 8-Methoxy-1,3,4,5-tetrahydro-2H-3-benzazepin-2-on

56,8 g (0,3 Mol) 8-Methoxy-1,3-dihydro-2H-3-benzazepin-2-on (Schmelzpunkt : 190-191 °C), gelöst in 600 ml Eisessig, werden in Anwesenheit von 5 g 10 %iger Palladiumkohle bei 80 °C und 5 bar 12 Stunden hydriert. Der Katalysator wird abgesaugt und die Essigsäure im Vakuum abdestilliert. Der Rückstand wird mit Wasser versetzt, mit Kaliumcarbonat neutralisiert, der ausgefallene Niederschlag abgesaugt, mit Wasser gewaschen und getrocknet.

Ausbeute : 51,1 g (89,1 % der Theorie),
Schmelzpunkt : 160-161 °C.

b) 7-Brom- und 9-Brom-8-methoxy-1,3,4,5-tetrahydro-2H-3-benzazepin-2-on

Zu 7,4 g (0,04 Mol) 8-Methoxy-1,3,4,5-tetrahydro-2H-3-benzazepin-2-on in 100 ml 80 %iger Essigsäure werden bei 3-5 °C unter Rühren 6,4 g = 2,03 ml (0,04 Mol) Brom in 10 ml Eisessig getropft. Nach 15 Minuten wird auf Eiswasser gegossen, mit Kaliumcarbonat neutralisiert, der Niederschlag abgesaugt, mit wenig Wasser gewaschen und getrocknet. Das erhaltene Isomerengemisch wird über eine Kieselgelsäule chromatographisch getrennt (Elutionsmittel : Essigester).

Ausbeute : 5,7 g (52,8 % der Theorie) 9-Brom-Isomeres
IR-Spektrum (Methylenchlorid) : 3 400 $cm^{-1}$ (NH) 1 660 $cm^{-1}$ (C = O)
4,1 g (39 % der Theorie) 7-Brom-Isomeres
IR-Spektrum (Kalciumbromid) : 3 220 $cm^{-1}$ (NH) 1 665 $cm^{-1}$ (CO)

c) 1-(7-Brom-8-methoxy-1,3,4,5-tetrahydro-2H-3-benzazepin-2-on-3-yl)-3-chlor-propan

Zu 1,35 g (5 mMol) 7-Brom-8-methoxy-1,3,4,5-tetrahydro-2H-3-benzazepin-2-on in 15 ml Dimethylsulfoxid werden 0,24 g (5,5 mMol) Natriumhydrid-Dispersion in Öl (55 %ig) zugesetzt und 1/2 Stunde bei Raumtemperatur und 10 Minuten bei 35-40 °C gerührt. Die Lösung wird zu 0,79 g (5,5 mMol) 1-Brom-3-chlorpropan in 5 ml Dimethylsulfoxid unter Rühren getropft. Anschließend rührt man 2 Stunden bei Raumtemperatur, gießt auf Eiswasser und extrahiert 4 mal mit Methylenchlorid. Die Methylenchlorid-Extrakte werden mehrmals mit Wasser gewaschen, getrocknet und im Vakuum eingeengt.

Der Rückstand wird über eine Kieselgelsäule mit Essigester als Elutionsmittel gereinigt.

Ausbeute : 210 mg (12 % der Theorie),

Schmelzpunkt : 119-120 °C.

Beispiel M

1-(7-Methoxy-1,3,4,5-tetrahydro-2H-3-benzazepin-2-on-3-yl)-3-chlor-propan

a) 7-Methoxy-1,3,4,5-tetrahydro-2H-3-benzazepin-2-on

3,1 g (0,0136 Mol) N-Chloracetyl-N-(2-(3-methoxy-phenyl)-ethyl)-amin werden in 270 ml Ethanol und 1 530 ml Wasser gelöst und 10 Stunden unter Stickstoffatmosphäre bei 20-25 °C mit einer Quecksilber-Hochdrucklampe belichtet. Die Lösung wird auf ein Volumen von ca. 400 ml eingeengt, mit Natriumbicarbonat versetzt und mehrmals mit Essigester ausgeschüttelt. Die Extrakte werden über Magnesiumsulfat getrocknet, eingeengt und der Rückstand über Magnesiumsulfat getrocknet, eingeengt und der Rückstand über eine Kieselgelsäule mit Essigester als Elutionsmittel gereinigt.

Ausbeute : 820 mg (31,5 % der Theorie),

Schmelzpunkt : 152-154 °C.

b) 1-(7-Methoxy-1,3,4,5-tetrahydro-2H-3-benzazepin-2-on-3-yl)-3-chlor-propan

1,15 g (6 mMol) 7-Methoxy-1,3,4,5-tetrahydro-2H-3-benzazepin-2-on werden in 30 ml absolutem Tetramethylharnstoff gelöst, mit 300 mg 55 %iger Natriumhydrid-Dispersion (in Öl) versetzt und unter einer Stickstoff-Atmosphäre 2 Stunden bei 20-25 °C gerührt. Das erhaltene Reaktionsgemisch wird unter Rühren bei 15-20 °C unter Stickstoff zu 1,6 g (7,8 mMol) 1-Chlor-3-jodpropan, gelöst in 20 ml Tetramethylharnstoff, getropft und 3 Stunden bei Raumtemperatur gerührt. Anschließend wird mit ca. 300 ml Essigester versetzt und 6 mal mit Wasser extrahiert. Die organische Lösung wird über Magnesiumsulfat getrocknet, eingeengt und der Rückstand über eine Kieselgelsäule mit Methylenchlorid und steigenden Anteilen Ethanol (bis 2 %) gereinigt.

Ausbeute : 410 mg (25,5 % der Theorie),

IR-Spektrum (Methylenchlorid) : 1 650 $cm^{-1}$ (CO).

Beispiel N

1-(7-Nitro-8-methoxy-1,3,4,5-tetrahydro-2H-3-benzazepin-2-on-3-yl)-3-chlor-propan

28,5 g (0,106 Mol) 1-(8-Methoxy-1,3,4,5-tetrahydro-2H-3-benzazepin-2-on-3-yl)-3-chlor-propan werden in 350 ml konzentrierter Salpetersäure 1/2 Stunde bei 20-25 °C gerührt. Die Lösung wird auf Eiswasser gegossen, mit Kaliumcarbonat neutralisiert und 2 mal mit Methylenchlorid extrahiert. Der Extrakt wird über Magnesiumsulfat getrocknet, im Vakuum eingeengt und der Rückstand über eine Kieselgelsäule mit Essigester als Elutionsmittel gereinigt.

Ausbeute : 11 g (33,2 % der Theorie),

Schmelzpunkt : 127-128 °C.

Beispiel O

1-[2-(2-Amino-4,5-dimethoxy-phenyl)-ethylamino]-3-[N-methyl-N-(2-(indolyl-3)-ethyl)-amino]-propan

a) 2-[N-Methyl-N-(2-cyano-ethyl)-amino]-ethyl-indol-3

23,4 g (0,135 Mol) 2-(N-Methyl)-aminoethyl-indol-3 in 200 ml absolutem Methanol und 8,9 ml (0,135 Mol) Acrylnitril werden 40 Minuten bei 50 °C gerührt. Nach dem Einengen in Vakuum wird über 800 g Aluminiumoxid (neutral, Aktivität II-III) mit Methylenchlorid und steigenden Anteilen von Ethanol (bis 3 %) als Elutionsmittel gereinigt.

Ausbeute : 20,8 g (67,8 % der Theorie),

IR-Spektrum (Methylenchlorid) : 2 240 $cm^{-1}$ CN

b) 1-Amino-3-[N-methyl-N-(2-(indolyl-3)-ethyl)-amino]-propan

7,6 g (0,033 Mol) 2-[N-Methyl-N-(2-cyanoethyl)-amino]-ethyl-indol-3 und 1,7 g Raney-Nickel in 70 ml mit Ammoniak gesättigtem Methanol werden 2 Stunden bei 50 °C und 5 bar hydriert. Nach Abfiltrieren des Katalysators und Einengen im Vakuum erhält man 7,7 g Amin.

Ausbeute : 7,7 g (100 % der Theorie)

c) 1-[2-(4,5-Dimethoxy-2-nitro-phenyl)-1-oxyethyl-amino]-3-[N-methyl-N-(2-(indolyl-3)-ethyl)-amino]-propan

Aus einer Suspension von 7,2 g (0,03 Mol) 4,5-Dimethoxy-2-nitro-phenylessigsäure in 80 ml absolutem Tetrahydrofuran wird durch Zugabe von 4,75 g (0,03 Mol) N,N'-Carbonyldiimidazol das Imidazolid dargestellt. Eine Lösung von 8,2 g (0,03 Mol) 1-Amino-3-[N-methyl-N-(2-(indolyl-3)-ethyl)-amino]-propan in 60 ml absolutem Tetrahydrofuran wird zugetropft und nach 2-stündigem Rühren bei Raumtemperatur wird eingeengt, in Methylenchlorid gelöst, mit 2 N Natronlauge extrahiert, mit Wasser gewaschen, getrocknet über Magnesiumsulfat, im Vakuum erneut eingeengt und über 800 g Aluminiumoxid (neutral, Aktivität II-III) mit Methylenchlorid und steigenden Anteilen von Ethanol (bis 4 %) gereinigt.
Ausbeute : 12,3 g (90,9 % der Theorie),
IR-Spektrum (Methylenchlorid) : 1 670 $cm^{-1}$ CO

d) 1-[2-(2-Amino-4,5-dimethoxy-phenyl)-1-oxoethyl-amino]-3-[N-methyl-N-(2-(indolyl-3)-ethyl)-amino]-propan

15 g (0,03 Mol) 1-[2-(4,5-Dimethoxy-2-nitro-phenyl)-1-oxoethyl-amino]-3-[N-methyl-N-(2-indolyl-3)-ethyl-amino]-propan werden in 160 ml Methanol gelöst, mit 0,8 g 10 %iger Palladium-Kohle versetzt und 9 Stunden bei Raumtemperatur und 5 bar hydriert. Nach Absaugen vom Katalysator wird im Vakuum eingeengt und über 1 200 g Aluminiumoxid (neutral, Aktivität II-III) mit Methylenchlorid und steigenden Anteilen von Ethanol (bis 12 %) gereinigt.
Ausbeute : 9,6 g (75,6 % der Theorie),
IR-Spektrum (Methylenchlorid) : 1 655 $cm^{-1}$ CO

e) 1-[2-(2-Amino-4,5-dimethoxy-phenyl)-ethyl-amino]-3-[N-methyl-N-(2-(indolyl-3)-ethyl)-amino]-propan

Zu einer 60 °C warmen Lösung von 2,13 g (0,005 Mol) 1-[2-(2-Amino-4,5-dimethoxy-phenyl)-1-oxoethyl-amino]-3-[N-methyl-N-(2-(indolyl-3)-ethyl)-amino]-propan in 35 ml absolutem Dioxan wird unter Stickstoff-Begasung eine Lösung von 4,3 ml (0,015 Mol) Natrium-bis(2-methoxyethoxy)-dihydroaluminat (70 %ige Lösung in Toluol, ca. 3,5 molar) in 4 ml absolutem Toluol langsam zugetropft und anschließend 4 Stunden am Rückfluß gekocht. Nach Zersetzung mit 1,8 ml 10 %iger Ammoniumchlorid-Lösung und 5 ml 2 molarer Natronlauge wird der Ansatz abfiltriert, eingeengt und über 120 g Aluminiumoxid (neutral, Aktivität II-III) mit Methylenchlorid und steigenden Anteilen an Ethanol (bis 3 %) als Elutionsmittel gereinigt.
Ausbeute : 2,05 g (100 % der Theorie).

## Beispiel P

1-(1,3,4,5-Tetrahydro-2H-3-benzazepin-2,4-dion-3-yl)-3-chlor-propan

a) 2-Amino-4-brom-1H-3-benzazepin-hydrobromid

Hergestellt aus 5,0 g (0,032 Mol) o-Phenylendiacetonitril analog Beispiel Fa.
Ausbeute : 8,0 g (78,6 % der Theorie)

b) 1,3,4,5-Tetrahydro-2H-3-benzazepin-2,4-dion

Hergestellt aus 8,0 g (0,025 Mol) 2-Amino-4-brom-1H-3-benzazepin-hydrobromid analog Beispiel Fb.
Ausbeute : 3,7 g (66,1 % der Theorie),
Schmelzpunkt : 189-191 °C

c) 1-(1,3,4,5-Tetrahydro-2H-3-benzazepin-2,4-dion-3-yl)-3-chlor-propan

3,5 g (0,020 Mol) 1,3,4,5-Tetrahydro-2H-3-benzazepin-2,4-dion werden in 30 ml Dimethylformamid suspendiert und unter Rühren mit 2,5 g Kalium-tert. butylat versetzt. Nach 10 Minuten wird die erhaltene Lösung unter Eiskühlung zu 3,5 ml 1-Brom-3-chlorpropan in 20 ml Dimethylformamid getropft. Nach einer Stunde gießt man auf Eiswasser. Nach kurzer Zeit kirstallisiert die schmierige Fällung. Der Niederschlag wird abgesaugt, in Aceton gelöst, nochmals mit Wasser gefällt, abgesaugt und getrocknet.
Ausbeute : 4,7 g (90,4 % der Theorie).

Herstellung der Endprodukte

## Beispiel 1

1-(7,8-Dimethoxy-1,3,4,5-tetrahydro-2H-3-benzazepin-2-on-3-yl)-3-[N-methyl-N-(2-(indolyl-3)-ethyl)-amino]-propan

2,98 g (0,01 Mol) 1-(7,8-Dimethoxy-1,3,4,5-tetrahydro-2H-3-benzazepin-2-on-3-yl)-3-chlor-propan, 1,39 ml (0,01 Mol) Triethylamin und 1,74 g (0,01 Mol) 3-(N-Methyl-2-amino-ethyl)-indol werden 4 Stunden bei 90 °C gerührt. Anschließend wird in Methylenchlorid gelöst, mit 1 %iger Essigsäure extrahiert, über Magnesiumsulfat getrocknet, einrotiert und über 150 g Kieselgelsäule mit Methylenchlorid und mit steigenden Anteilen von Ethanol (bis 10 %) gereinigt.

Ausbeute : 1,69 g (38,8 % der Theorie),
Schmelzpunkt : 136-140 °C.

## Beispiel 2

1-(7,8-Dimethoxy-1,3,4,5-tetrahydro-2H-3-benzazepin-1,2-dion-3-yl)-3-[N-methyl-N-(2-(indolyl-3)-ethyl)-amino]-propan

0,41 g (0,0036 Mol) Selendioxid werben bei 70 °C in einer Mischung aus 17 ml Dioxan und 0,7 ml Wasser gegeben, 15 Minuten gerührt und dann mit 0,34 g Kieselgur und 1,5 g (0,034 Mol) 1-(7,8-Dimethoxy-1,3,4,5-tetrahydro-2H-3-benzazepin-2-on-3-yl)-3-[N-methyl-N-(2-(indolyl-3)-ethyl)-amino]-propan versetzt. Das Gemisch wird 46 Stunden unter Rückfluß gekocht, abgekühlt und filtriert. Das Filtrat wird im Vakuum eingeengt und der Rückstand über eine Kieselgel-Säule mit Methylenchlorid mit steigendem Anteil Ethanol (bis 20 %) als Elutionsmittel gereinigt.

Ausbeute : 0,15 g (10 % der Theorie),
Schmelzpunkt : 55-59 °C

## Beispiel 3

1-(7,8-Dimethoxy-1,3,4,5-tetrahydro-2H-3-benzazepin-2-on-3-yl)-3-[N-methyl-N-(2-(5-methoxy-indolyl-3)-ethyl)-amino]-propan

Hergestellt analog Beispiel 1 durch Umsetzung von 1-(7,8-Dimethoxy-1,3,4,5-tetrahydro-2H-3-benzazepin-2-on-3-yl)-3-chlor-propan mit 5-Methoxy-3-(N-methyl-2-amino-ethyl)-indol.

Ausbeute : 23,7 % der Theorie,
Schmelzpunkt : 55-59 °C.

## Beispiel 4

1-(7,8-Dimethoxy-1,3,4,5-tetrahydro-2H-3-benzazepin-2-on-3-yl)-3-[N-(2-(indolyl-3)-ethyl)-amino]-propan

0,6 g (0,002 Mol) 1-(7,8-Dimethoxy-1,3,4,5-tetrahydro-2H-3-benzazepin-2-on-3-yl)-3-chlor-propan und 3,2 g (0,02 Mol) Tryptamin werden eine Stunde auf 130 °C erhitzt. Nach Extraktion mit Essigester/Wasser wird die organische Phase über Magnesiumsulfat getrocknet, in Vakuum eingeengt und zweimal über 100 g Aluminiumoxid (neutral, Aktivität II) mit Methylenchlorid und steigenden Anteilen von Ethanol (bis 50 %) als Elutionsmittel gereinigt.

Ausbeute : 0,32 g (38 % der Theorie),
Schmelzpunkt : 66-67 °C.

## Beispiel 5

1-(7,8-Dimethoxy-1,3,4,5-tetrahydro-2H-3-benzazepin-2-thion-3-yl)-3-[N-methyl-N-(2-(indolyl-3)-ethyl)-amino]-propan

2,18 g (0,005 Mol) 1-(7,8-Dimethoxy-1,3,4,5-tetrahydro-2H-3-benzazepin-2-on-3-yl)-3-[N-methyl-N-(2-(indolyl-3)-ethyl)-amino]-propan und 1,5 g (0,0037 Mol) 2,4-Bis(3-methoxy-phenyl)-1,3-dithia-2,4-diphosphetan-2,4-disulfid werden in 10 ml Toluol suspendiert und 3 Stunden unter Rückfluß gekocht. Anschließend wird einrotiert und über 400 g Aluminiumoxid (neutral, Aktivität II) mit Methylenchlorid und mit steigenden Anteilen von Ethanol (bis 5 %) gereinigt.

Ausbeute : 0,65 g (28,8 % der Theorie),
Schmelzpunkt : 66-71 °C.

## Beispiel 6

1-(1-Hydroxy-7,8-dimethoxy-1,3,4,5-tetrahydro-2H-3-benzazepin-2-on-3-yl)-3-[N-methyl-N-(2-(indolyl-3)-ethyl)-amino]-propan

0,15 g (0,00033 Mol) 1-(7,8-Dimethoxy-1,3,4,5-tetrahydro-2H-3-benzazepin-1,2-dion-3-yl)-3-[N-methyl-N-(2-(indolyl-3)-ethyl)-amino]-propan werden in einem Gemisch aus Methanol/-Wasser = 95 : 5 gelöst, mit 0,015 g (0,0004 Mol) Natriumborhydrid versetzt und bei Raumtemperatur 15 Minuten gerührt. Danach wird mit 2N Salzsäure angesäuert, mit methanolischem Ammoniak alkalisch gestellt und filtriert. Das Filtrat wird eingeengt, in Methylenchlorid aufgenommen, über Magnesiumsulfat getrocknet und nochmals eingeengt.

Ausbeute : 9,11 g (73,3 % der Theorie),
Öl, Rf-Wert : 0,24 (Kieselgel, Methylenchlorid + 10 % Ethanol)
Ber. : C 69,16 H 7,37 N 9,31
Gef. : C 68,98 H 7,20 N 9,25

## Beispiel 7

1-(7,8-Dimethoxy-2,3,4,5-tetrahydro-1H-3-benzazepin-3-yl)-3-[N-methyl-N-(2-(indolyl-3)-ethyl)-amino]-propan

2,18 g (0,005 Mol) 1-(7,8-Dimethoxy-1,3,4,5-tetrahydro-2H-3-benzazepin-2-on-3-yl)-3-[N-methyl-N-(2-(indolyl-3)-ethyl)-amino]-propan werden in 30 ml Ether suspendiert und diese Suspension zu einer Suspension von 0,75 g (0,02 Mol) Lithiumaluminiumhydrid in 50 ml Ether getropft. Danach wird 8 Stunden unter Rückfluß gekocht, anschließend mit Wasser und 15 %iger Natronlauge vorsichtigt zersetzt und abgesaugt. Der Niederschlag wird mit Ethanol gewaschen, getrocknet und über 300 g Aluminiumoxid (neutral, Aktivität II) mit Methylenchlorid gereinigt.

Ausbeute : 0,67 g (31,9 % der Theorie),
Öl, Rf-Wert : 0,78 (Aluminiumoxid neutral, Methylenchlorid + 10 % Ethanol)
Ber. : C 74,07 H 8,37 N 9,97
Gef. : C 73,70 H 8,63 N 9,62

## Beispiel 8

1-(7,8-Dimethoxy-1,3,4,5-tetrahydro-2H-1,3-benzodiazepin-2-on-3-yl)-3-[N-methyl-N-(2-(indolyl-3)-ethyl)-amino]-propan

2,0 g (0,005 Mol) 1-[2-(2-Amino-4,5-dimethoxy-phenyl)-ethyl-amino]-3-[N-methyl-N-(2-(indolyl-3)-ethyl)-amino]-propan und 1,0 g (0,006 Mol), N,N'-Carbonyldiimidazol werden 60 Minuten in 40 ml absolutem Essigsäureäthylester unter Rückfluß gekocht. Nach Extraktion mit gesättigter Kaliumcarbonat-Lösung und mit Wasser wird das im Vakuum eingeengte Produkt in Methylenchlorid gelöst und mit Petroläther ausgefällt.

Ausbeute : 0,57 g (26,1 % der Theorie),
Schmelzpunkt : 168-169 °C.

## Beispiel 9

1-(7,8-Dimethoxy-1,3,4,5-tetrahydro-2H-3-benzazepin-2-on-3-yl)-3-[N-methyl-N-(2-(N-methyl-indolyl-3)-ethyl)-amino]-propan

Hergestellt analog Beispiel 1 durch Umsetzung von 1-(7,8-Dimethoxy-1,3,4,5-tetrahydro-2H-3-benzazepin-2-on-3-yl)-3-chlor-propan mit N-Methyl-3-(N-methyl-2-amino-ethyl)-indol.

Ausbeute : 18,0 % der Theorie,
Öl, Rf-Wert : 0,37 (Kieselgel, Methylenchlorid + 10 % Ethanol)
Ber. : C 72,13 H 7,85 N 9,35
Gef. : C 71,92 H 7,80 N 9,22

## Beispiel 10

1-(7,8-Dimethoxy-1,3,4,5-tetrahydro-2H-3-benzazepin-2-on-3-yl)-3-[N-methyl-N-(2-(5-benzyloxy-indolyl-3)-ethyl)-amino]-propan

Hergestellt analog Beispiel 1 durch Umsetzung von 1-(7,8-Dimethoxy-1,3,4,5-tetrahydro-2H-3-benzazepin-2-on-3-yl)-3-chlor-propan mit 5-Benzyloxy-3-(N-methyl-2-amino-ethyl)-indol.

Ausbeute : 10,4 % der Theorie,
Öl, Rf-Wert : 0,46 (Aluminiumoxid neutral, Methylenchlorid + 5 % Ethanol)
Ber. : C 73,17 H 7,26 N 7,76
Gef. : C 72,77 H 7,06 N 7,70

## Beispiel 11

1-(7,8-Dimethoxy-1,3,4,5-tetrahydro-2H-3-benzazepin-2-on-3-yl)-3-[N-methyl-N-(2-(5-hydroxy-indolyl-3)-ethyl)-amino]-propan

Hergestellt analog Beispiel 1 durch Umsetzung von 1-(7,8-Dimethoxy-1,3,4,5-tetrahydro-2H-3-benzazepin-2-on-3-yl)-3-chlor-propan mit 5-Hydroxy-3-(N-methyl-2-amino-ethyl)-indol.
Ausbeute : 56,0 % der Theorie,
Schmelzpunkt : 68-75 °C.

Beispiel 12

1-(7,8-Dimethoxy-1,3,4,5-tetrahydro-2H-3-benzazepin-2-on-3-yl)-3-[N-methyl-N-(3-(indolyl-3-)-propyl)-amino]-propan

Hergestellt analog Beispiel 1 durch Umsetzung von 1-(7,8-Dimethoxy-1,3,4,5-tetrahydro-2H-3-benzazepin-2-on-3-yl)-3-chlor-propan mit 3-(N-Methyl-3-amino-propyl)-indol.
Ausbeute : 36,2 % der Theorie,
Schmelzpunkt : 49-54 °C.

Beispiel 13

1-(7,8-Dimethoxy-1,3,4,5-tetrahydro-2H-3-benzazepin-2-on-3-yl)-3-[N-methyl-N-(2-(5-brom-indolyl-3)-ethyl)-amino]-propan

Hergestellt analog Beispiel 1 durch Umsetzung von 1-(7,8-Dimethoxy-1,3,4,5-tetrahydro-2H-3-benzazepin-2-on-3-yl)-3-chlor-propan mit 5-Brom-3-(N-methyl-2-amino-ethyl)-indol.
Ausbeute : 7,9 % der Theorie,
Schmelzpunkt : 74-80 °C.

Beispiel 14

1-(7,8-Dimethoxy-1,3,4,5-tetrahydro-2H-3-benzazepin-2-on-3-yl)-3-[N-methyl-N-(2-(7-methyl-indolyl-3)-ethyl)-amino]-propan

Hergestellt analog Beispiel 1 durch Umsetzung von 1-(7,8-Dimethoxy-1,3,4,5-tetrahydro-2H-3-benzazepin-2-on-3-yl)-3-chlor-propan mit 7-Methyl-3-(N-methyl-2-amino-ethyl)-indol.
Ausbeute : 15,6 % der Theorie,
Schmelzpunkt : 62-66 °C.

Beispiel 15

1-(7,8-Dimethoxy-1,3-dihydro-2H-3-benzazepin-2-on-3-yl)-3-[N-methyl-N-(2-(indolyl-3)-ethyl)-amino]-propan

Hergestellt analog Beispiel 1 durch Umsetzung von 1-(7,8-Dimethoxy-1,3-dihydro-2H-3-benzazepin-2-on-3-yl)-3-chlor-propan mit 3-(N-Methyl-2-amino-ethyl)-indol.
Ausbeute : 17,7 % der Theorie,
Schmelzpunkt : 64-70 °C.

Beispiel 16

1-(1,3,4,5-Tetrahydro-2H-3-benzazepin-2,4-dion-3-yl)-3-[N-methyl-N-(2-(indolyl-3)-ethyl)-amino]-propan-hydrochlorid

Hergestellt analog Beispiel 1 durch Umsetzung von 1-(1,3,4,-5-Tetrahydro-2H-3-benzazepin-2,4-dion-3-yl)-3-chlor-propan mit 3-(N-Methyl-2-amino-ethyl)-indol und anschließender Fällung des Hydrochlorids mit etherischer Salzsäure.
Ausbeute : 73,9 % der Theorie,
Schmelzpunkt : 91-95 °C.

Beispiel 17

1-(7,8-Dichlor-1,3,4,5-tetrahydro-2H-1,3-benzodiazepin-2-on-3-yl)-3-[N-methyl-N-(2-(N-methyl-5-benzyloxy-indolyl-3)-ethyl)-amino]-propan

Hergestellt analog Beispiel 8 durch Umsetzung von 1-[2-(2-Amino-4,5-dichlor-phenyl)-ethyl-amino]-3-[N-methyl-N-(2-(N-methyl-5-benzyloxy-indolyl-3)-ethyl)-amino]-propan und N,N'-Carbonyl-diimidazol.

Ausbeute : 37,4 % der Theorie,

Öl, Rf-Wert : 0,38 (Aluminiumoxid neutral, Methylenchlorid + 5 % Ethanol)

Ber. : C 65,84 H 6,06 N 9,91 Cl 12,54

Gef. : C 65,46 H 6,00 N 9,58 Cl 12,32

Analog den vorstehenden Beispielen werden folgende Verbindungen erhalten :

1-(7,8-Dimethoxy-2,3-dihydro-1H-3-benzazepin-3-yl)-3-[N-methyl-N-(2-(indolyl-3)-ethyl)-amino]-propan

1-(7,8-Methylendioxy-1,3,4,5-tetrahydro-2H-3-benzazepin-2-on-3-yl)-3-[N-methyl-N-(2-(indolyl-3-)-ethyl)-amino]-propan

1-(7,8-Dimethyl-1,3,4,5-tetrahydro-2H-3-benzazepin-2-on-3-yl)-3-[N-methyl-N-(2-(indolyl-3)-ethyl)-amino]-propan

1-(7,8-Dichlor-1,3,4,5-tetrahydro-2H-3-benzazepin-2-on-3-yl)-3-[N-methyl-N-(2-(indolyl-3)-ethyl)-amino]-propan

1-(7-Brom-8-methoxy-1,3,4,5-tetrahydro-2H-3-benzazepin-2-on-3-yl)-3-[N-methyl-N-(2-(indolyl-3)-ethyl)-amino]-propan

1-(7-Dimethylamino-8-methoxy-1,3,4,5-tetrahydro-2H-3-benzazepin-2-on-3-yl)-3-[N-methyl-N-(2-(indolyl-3)-ethyl)-amino]-propan

1-(7-Methoxy-1,3,4,5-tetrahydro-2H-3-benzazepin-2-on-3-yl)-3-[N-methyl-N-(2-(indolyl-3)-ethyl)-amino]-propan

1-(7-Nitro-8-methoxy-1,3,4,5-tetrahydro-2H-3-benzazepin-2-on-3-yl)-3-[N-methyl-N-(2-(indolyl-3)-ethyl)-amino]-propan

1-(1,3,4,5-Tetrahydro-2H-3-benzazepin-2-on-3-yl)-3-[N-methyl-N-(2-Indolyl-3)-ethyl)-amino]-propan

## Beispiel I

Tabletten zu 10 mg 1-(7,8-Dimethoxy-1,3,4,5-tetrahydro-2H-3-benzazepin-2-on-3-yl)-3-[N-methyl-N-(2-(indolyl-3)-ethyl)-amino]-propan

Zusammensetzung :

1 Tablette enthält :

| | |
|---|---|
| Wirksubstanz | 10,0 mg |
| Maisstärke | 57,0 mg |
| Milchzucker | 48,0 mg |
| Polyvinylpyrrolidon | 4,0 mg |
| Magnesiumstearat | 1,0 mg |
| | 120,0 mg |

Herstellungsverfahren

Der Wirkstoff, Maisstärke, Milchzucker und Polyvinylpyrrolidon werden gemischt und mit Wasser befeuchtet. Die feuchte Mischung wird durch ein Sieb mit 1,5 mm-Maschenweite gedrückt und bei ca. 45 °C getrocknet. Das trockene Granulat wird durch ein Sieb mit 1,0 mm-Maschenweite geschlagen und mit Magnesiumstearat vermischt. Die fertige Mischung preßt man auf einer Tablettenpresse mit Stempeln von 7 mm Durchmesser, die mit einer Teilkerbe versehen sind, zu Tabletten. Tablettengewicht : 120 mg

## Beispiel II

Dragees zu 5 mg 1-(7,8-Dimethoxy-1,3,4,5-tetrahydro-2H-3-benzazepin-2-on-3-yl)-3-[N-methyl-N-(2-(indolyl-3)-ethyl)-amino]-propan

1 Dragéekern enthält :

| | |
|---|---|
| Wirksubstanz | 5,0 mg |
| Maisstärke | 41,5 mg |
| Milchzucker | 30,0 mg |
| Polyvinylpyrrolidon | 3,0 mg |
| Magnesiumstearat | 0,5 mg |
| | 80,0 mg |

Herstellungsverfahren

Der Wirkstoff, Maisstärke, Milchzucker und Polyvinylpyrrolidon werden gut gemischt und mit Wasser befeuchtet. Die feuchte Masse drückt man durch ein Sieb mit 1 mm-Maschenweite, trocknet bei ca. 45 °C und schlägt das Granulat anschließend durch dasselbe Sieb. Nach dem Zumischen von Magnesiumstearat werden auf einer Tablettiermaschine gewölbte Drageekerne mit einem Durchmesser von 6 mm gepreßt. Die so hergestellten Dragéerkerne werden auf bekannte Weise mit einer Schicht Überzogen, die im wesentlichen aus Zucker und Talkum besteht. Die fertigen Dragées werden mit Wachs poliert.

Dragéegewicht : 130 mg

Beispiel III

Amullen zu 5 mg 1-(7,8-Dimethoxy-1,3,4,5-tetrahydro-2H-3-benzazepin-2-on-3-yl)-3-[N-methyl-N-(2-(indolyl-3)-ethyl)-amino]-propan

1 Ampulle enthält :

| | |
|---|---|
| Wirksubstanz | 5,0 mg |
| Sorbit | 50,0 mg |
| Wasser für Injektionszwecke ad | 2,0 ml |

Herstellungsverfahren

In einem geeigneten Ansatzgefäß wird der Wirkstoff in Wasser für Injektionszwecke gelöst und die Lösung mit Sorbit isotonisch gestellt.

Nach Filtration über einen Membranfilter wird die Lösung unter $N_2$-Begasung in gereinigte und sterilisierte Ampullen abgefüllt und 20 Minuten im strömenden Wasserdampf autoklaviert.

Beispiel IV

Suppositorien zu 15 mg 1-(7,8-Dimethoxy-1,3,4,5-tetrahydro-2H-3-benzazepin-2-on-3-yl)-3-[N-methyl-N-(2-(indolyl-3)-ethyl)-amino]-propan

1 Zäpfchen enthält :

| | |
|---|---|
| Wirksubstanz | 0,015 g |
| Hartfett (z. B. Witepsol H 19 und W 45) | 1,685 g |
| | 1,700 g |

Herstellungsverfahren

Das Hartfett wird geschmolzen. Bei 38 °C wird die gemahlene Wirksubstanz in der Schmelze homogen dispergiert. Es wird auf 35 °C abgekühlt und in schwach vorgekühlte Suppositorienformen ausgegossen.

Beispiel V

Tropfenlösung mit 10 mg 1-(7,8-Dimethoxy-1,3,4,5-tetrahydro-2H-3-benzazepin-2-on-3-yl)-3-[N-methyl-N-(2-(indolyl-3)-ethyl)-amino]-propan

100 ml Lösungen enthalten :

| | |
|---|---|
| Wirksubstanz | 0,2 g |
| Hydroxyäthylcellulose | 0,15 g |
| Weinsäure | 0,1 g |
| Sorbitlösung 70 % Trockensubstanz | 30,0 g |
| Glycerin | 10,0 g |
| Benzoesäure | 0,15 g |
| Dest. Wasser ad | 100 ml |

Herstellungsverfahren

Dest. Wasser wird auf 70 °C erhitzt. Hierin wird unter Rühren Hydroxyäthylcellulose, Benzoesäure und Weinsäure gelöst. Es wird auf Raumtemperatur abgekühlt und hierbei das Glycerin und die Sorbitlösung unter Rühren zugegeben. Bei Raumtemperatur wird der Wirkstoff zugegeben und bis zur völligen Auflösung gerührt. Anschließend wird zur Entlüftung des Saftes unter Rühren evakuiert.

**Patentansprüche**

1. Indolderivate der allgemeinen Formel I

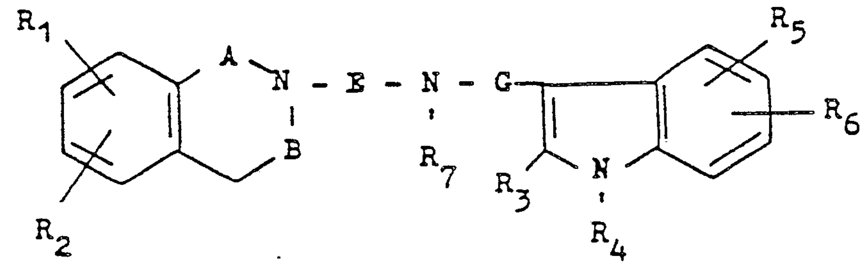

(I)

in der

A eine $-CH_2-CH_2-$, $-CH=CH-$, $-\underset{x}{NH}-CO-$ oder $-\underset{x}{CH_2}-CO$-Gruppe

und

B eine Methylen-, Carbonyl- oder Thiocarbonylgruppe oder

A eine oder $-\underset{x}{\overset{OH}{|}{CH}}-CO$

Gruppe und B eine Methylengruppe, wobei das mit x gekennzeichnete Kohlenstoffatom jeweils mit dem Phenylkern verknüpft ist,

E eine gegebenenfalls durch eine Alkylgruppe mit 1 bis 3 Kohlenstoffatomen substituierte geradkettige Alkylengruppe mit 2 bis 4 Kohlenstoffatomen, eine 2-Hydroxy-n-propylen-, 2-Hydroxy-n-butylen- oder 3-Hydroxy-n-butylengruppe, G eine gegebenenfalls durch eine Alkylgruppe mit 1 bis 3 Kohlenstoffatomen substituierte geradkettige Alkylengruppe mit 1 bis 5 Kohlenstoffatomen,

$R_1$ ein Wasserstoff-, Chlor- oder Bromatom, eine Trifluormethyl-, Nitro-, Amino-, Alkylamino-, Dialkylamino-, Alkyl-, Hydroxy-, Alkoxy- oder Phenylalkoxygruppe, wobei jeder Alkylteil jeweils 1 bis 3 Kohlenstoffatome enthalten kann,

$R_2$ ein Wasserstoff-, Chlor- oder Bromatom, eine Hydroxy-, Alkoxy-, Phenylalkoxy- oder Alkylgruppe, wobei jeder Alkylteil jeweils 1 bis 3 Kohlenstoffatome enthalten kann, oder

$R_1$ und $R_2$ zusammen eine Alkylendioxygruppe mit 1 bis 2 Kohlenstoffatomen,

$R_3$ ein Wasserstoff-, Chlor- oder Bromatom oder eine Alkylgruppe mit 1 bis 4 Kohlenstoffatomen,

$R_4$ ein Wasserstoffatom, eine Alkyl- oder Phenylalkylgruppe, wobei der Alkylteil jeweils 1 bis 3 Kohlenstoffatome enthalten kann,

$R_5$ ein Wasserstoff-, Fluor-, Chlor- oder Bromatom, eine Alkyl-, Hydroxy-, Alkoxy- oder Phenylalkoxygruppe, wobei jeder Alkylteil jeweils 1 bis 3 Kohlenstoffatome enthalten kann,

$R_6$ ein Wasserstoffatom oder eine Alkoxygruppe mit 1 bis 3 Kohlenstoffatomen und

$R_7$ ein Wasserstoffatom, eine Alkenylgruppe mit 3 bis 5 Kohlenstoffatomen, eine Alkyl- oder Phenylalkylgruppe, wobei der Alkylteil jeweils 1 bis 3 Kohlenstoffatomen enthalten kann, bedeuten, und deren Säureadditionssalze.

2. Indolderivate der allgemeinen Formel Ia gemäß Anspruch 1

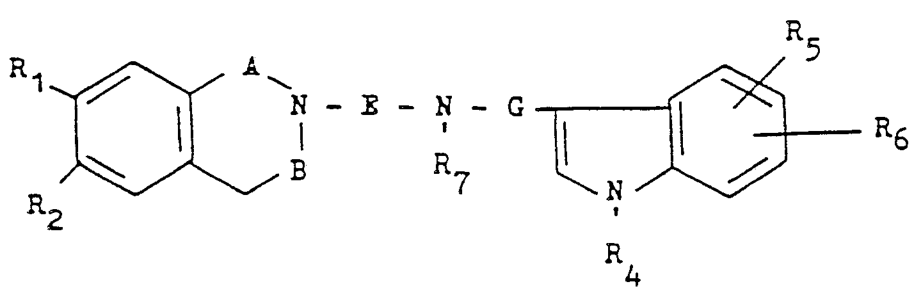

(Ia)

in der

A und B wie im Anspruch 1 definiert sind,

E eine n-Propylengruppe,

G eine Ethylen- oder n-Propylengruppe,

$R_1$ ein Chlor- oder Bromatom, eine Methyl-, Methoxy-, Nitro-, Amino-, Methylamino- oder Dimethylaminogruppe,

$R_2$ ein Wasserstoff-, Chlor- oder Bromatom, eine Methoxygruppe oder $R_1$ und $R_2$ zusammen eine Methylendioxygruppe,

$R_4$ ein Wasserstoffatom oder eine Methylgruppe,

$R_5$ ein Wasserstoff-, Fluor-, Chlor- oder Bromatom, eine Methyl-, Hydroxy-, Methoxy- oder Benzyloxygruppe,

$R_6$ ein Wasserstoffatom oder eine Methoxygruppe und

$R_7$ ein Wasserstoffatom oder eine Methylgruppe bedeuten, und deren Säureadditionssalze.

3. Indolderivate der allgemeinen Formel Ia gemäß Anspruch 2, in der

A eine $—CH_2CH_2—$ oder —CH—CH-Gruppe und B eine Carbonylgruppe oder

A eine —COCO— der —NH—CO-Gruppe und B eine Methylengruppe,

E eine n-Propylengruppe,

G eine Ethylengruppe,

$R_1$ und $R_2$ je eine Methoxygruppe,

$R_4$, $R_5$ und $R_6$ je ein Wasserstoffatom und

$R_7$ eine Methylgruppe bedeuten, und deren Säureadditionssalze.

4. 1-(7,8-Dimethoxy-1,3,4,5-tetrahydro-2H-3-benzazepin-2-on-3-yl)-3-[N-methyl-N-(2-(indolyl-3)-ethyl)-amino]-propan und dessen Säureadditionssalze.

5. 1-(7,8-Dimethoxy-1,3,4,5-tetrahydro-2H-3-benzazepin-1,2-dion-3-yl)-3-[N-methyl-N-(2-(indolyl-3)-ethyl)-amino]-propan und dessen Säureadditionssalze.

6. Physiologisch verträgliche Säureadditionssalze der Verbindungen gemäß den Ansprüchen 1 bis 5 mit anorganischen oder organischen Säuren.

7. Arzneimittel, enthaltend eine Verbindung der allgemeinen Formel I gemäß den Ansprüchen 1 bis 5 oder deren physiologisch verträgliches Säureadditionssalz gemäß Anspruch 6 neben einem oder mehreren inerten Trägerstoffen und/oder Verdünnungsmitteln.

8. Verfahren zur Herstellung eines Arzneimittels zur Behandlung von Sinustachykardien und ischämischer Herzerkrankungen, dadurch gekennzeichnet, daß auf nichtchemischem Wege eine Verbindung gemäß den Ansprüchen 1 bis 5 oder deren physiologisch verträgliches Säureadditionsalz gemäß Anspruch 6 in einen oder mehrere inerte Trägerstoffe und/oder Verdünnungsmittel eingearbeitet wird.

9. Verwendung einer Verbindung gemäß den Ansprüchen 1 bis 6 zur Herstellung eines Arzneimittels, welches zur Behandlung von Sinustachykardien und ischämischen Herzerkrankungen geeignet ist.

10. Verfahren zur Herstellung von neuen Indolderivaten gemäß den Ansprüchen 1 bis 6, dadurch gekennzeichnet, daß

a) eine Verbindung der allgemeinen Formel II

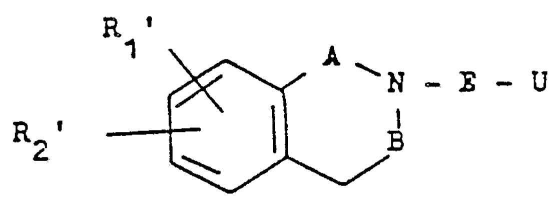

(II)

mit einer Verbindung der allgemeinen Formel III

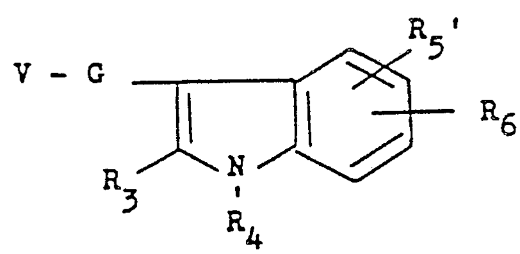

(III)

in der

$R_3$, $R_4$, $R_6$, A, B, E und G wie eingangs definiert sind,

$R_1$ eine durch einen Schutzrest geschützte Hydroxy-, Amino- oder Alkylaminogruppe darstellt oder die für $R_1$ eingangs erwähnten Bedeutungen besitzt,

$R_2'$ eine durch einen Schutzrest geschützte Hydroxygruppe darstellt oder die für $R_2$ eingangs erwähnten Bedeutungen besitzt,

$R_5'$ eine durch einen Schutzrest geschützte Hydroxygruppe darstellt oder die für $R_5$ eingangs erwähnten Bedeutungen besitzt, einer der Reste U oder V die $R_7'$ —NH-Gruppe, wobei $R_7'$ eine Schutzgruppe für eine Aminogruppe darstellt oder die

für $R_7'$ eingangs erwähnten Bedeutungen besitzt, und der andere der Reste U oder V eine nukleophile Austrittsgruppe wie ein Halogenatom oder eine Sulfonyloxygruppe darstellt, umgesetzt und gegebenenfalls anschließend ein verwendeter Schutzrest abgespalten wird oder

b) zur Herstellung von Verbindungen der allgemeinen Formel I, in der B eine Thiocarbonylgruppe darstellt, eine Verbindung der allgemeinen Formel IV

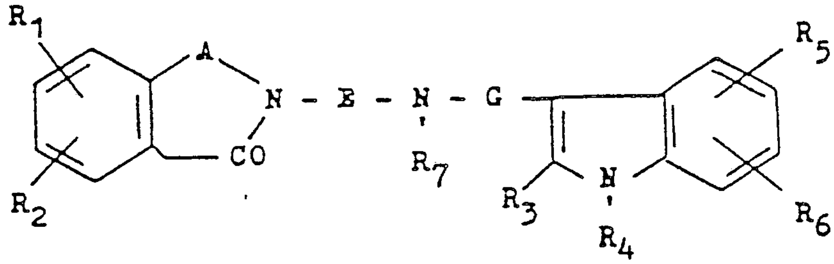

(IV)

in der $R_1$ bis $R_7$, A, E und G wie eingangs definiert sind, mit einem schwefeleinführenden Mittel umgesetzt wird oder

c) zur Herstellung von Verbindungen der allgemeinen Formel I,

in der A eine

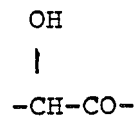

Gruppe darstellt, eine Verbindung der allgemeinen Formel V

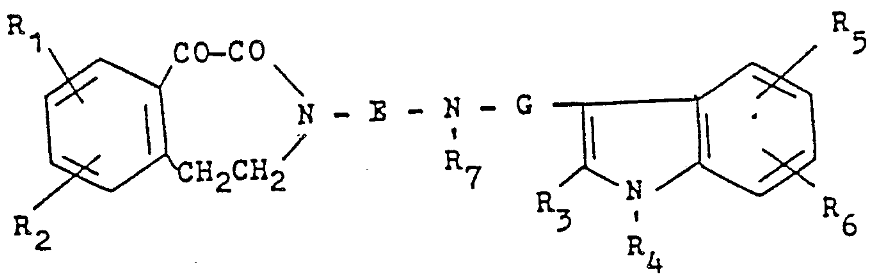

(V)

in der $R_1$ bis $R_7$, E und G wie eingangs definiert sind, reduziert wird oder

d) zur Herstellung von Verbindungen der allgemeinen Formel I, in der A eine $-CH_2-CH_2-$ oder $-CH=CH-$ und B eine Methylengruppe darstellen, eine Verbindung der allgemeinen Formel VI

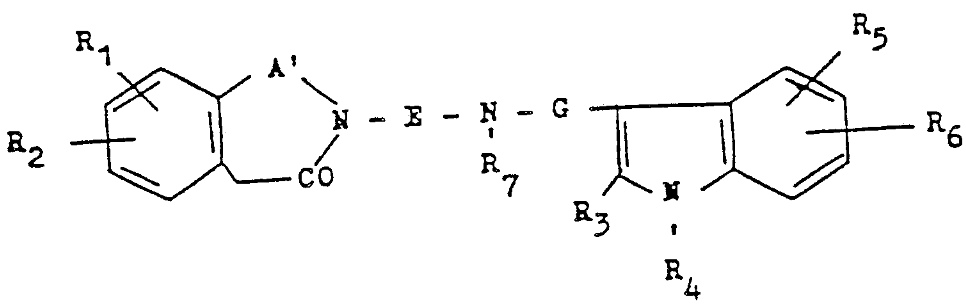

(VI)

in der

$R_1$ bis $R_7$, E und G wie eingangs definiert sind und

A' eine $-CH_2-CH_2-$ oder $-CH=CH$-Gruppe darstellt, reduziert wird oder

e) zur Herstellung von Verbindungen der allgemeinen Formel I, in der A die $-COCO$-Gruppe darstellt, eine Verbindung der allgemeinen Formel VII

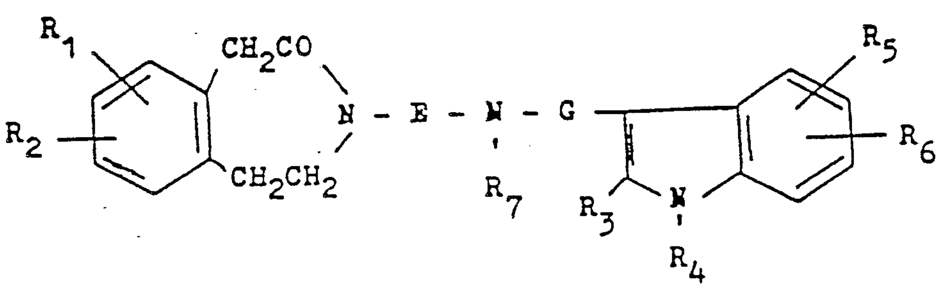

(VII)

in der $R_1$ bis $R_7$, E und G eingangs definiert sind, oxidiert wird oder

f) zur Herstellung von Verbindungen der allgemeinen Formel I, in der A die —NH—CO-Gruppe und E eine gegebenenfalls durch eine Alkylgruppe mit 1 bis 3 Kohlenstoffatomen subsituierte Alkylengruppe mit 2 bis 4 Kohlenstoffatomen darstellt, eine Verbindung der allgemeinen Formel VIII

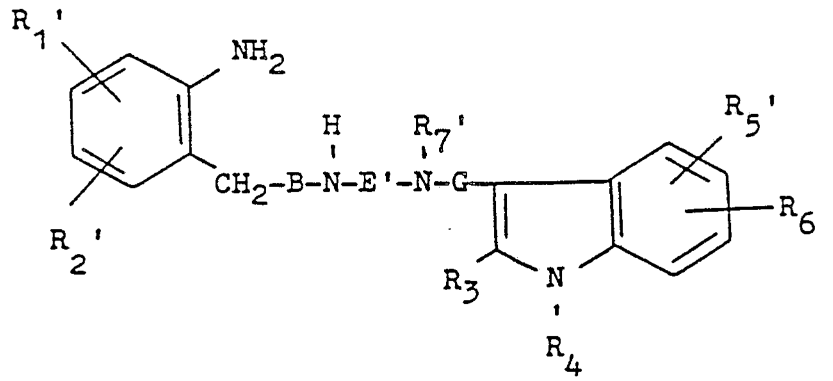

(VIII)

in der

B, G, $R_3$, $R_4$ und $R_6$ wie eingangs definiert sind,

E' eine gegebenenfalls durch eine Alkylgruppe mit 1 bis 3 Kohlenstoffatomen substituierte Alkenylgruppe mit 2 bis 4 Kohlenstoffatomen,

$R_1'$ eine durch eine Schutzgruppe geschützte Hydroxy-, Amino- der Alkylaminogruppe darstellt oder die für $R_1$ eingangs erwähnten Bedeutungen besitzt,

$R_2'$ eine durch eine Schutzgruppe geschützte Hydroxygruppe darstellt oder die für $R_2$ eingangs erwähnten Bedeutungen besitzt,

$R_5'$ eine durch eine Schutzgruppe geschützte Hydroxygruppe darstellt oder die für $R_5$ eingangs erwähnten Bedeutungen besitzt,

$R_7'$ eine Schutzgruppe für eine Aminogruppe darstellt oder mit Ausnahme von Wasserstoff die für $R_7$ eingangs erwähnten Bedeutungen besitzt, mit einem Kohlensäurederivat der allgemeinen Formel IX

$$W—CO—W \qquad (IX)$$

in der W, die gleich oder verschieden sein können, jeweils eine nukleophile Austrittsgruppe bedeuten, umgesetzt und gegebenenfalls anschließend ein verwendeter Schutzrest abgespalten wird und gewünschtenfalls anschließend eine so erhaltene Verbindung der allgemeinen Formel I in ihr Säureadditionssalz, insbesondere in ihr physiologisch verträgliches Säureadditionssalz mit einer anorganischen oder organischen Säure übergeführt wird.

## Claims

1. Indole derivates of general formula I

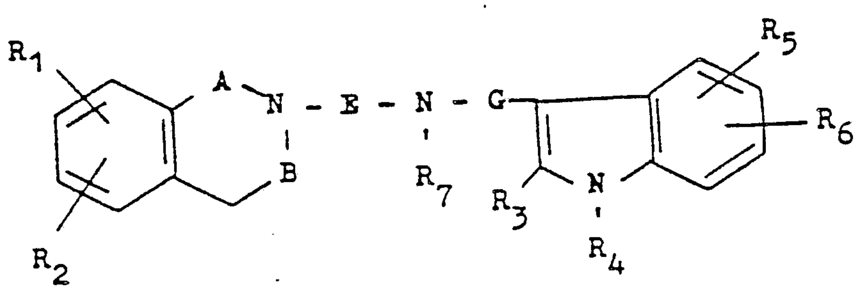

(I)

wherein

A represents a $—CH_2—CH_2$, —CH=CH, $-\underset{x}{NH}-CO$ or $-\underset{x}{CH_2}-CO$ group

and

B represents a methylene, carbonyl or thiocarbonyl group or

A represents a CO—CO or $-\underset{x}{\overset{OH}{CH}}-CO$

group and B represents a methylene group, wherein the carbon atom designated x is linked to the phenyl nucleus in each case,

E represents a straight-chained alkylene group with 2 to 4 carbon atoms optionally substituted by an alkyl group with 1 to 3 carbon atoms, a 2-hydroxy-n-propylene, 2-hydroxy-n-butylene or 3-hydroxy-n-butylene group,

G represents a straight-chained alkylene group with 1 to 5 carbon atoms optionally substituted by an alkyl group with 1 to 3 carbon atoms,

$R_1$ represents a hydrogen, chlorine or bromine atom, a trifluoromethyl, nitro, amino, alkylamino, dialkylamino, alkyl, hydroxy, alkoxy or phenylalkoxy group, whilst each alkyl moiety may contain from 1 to 3 carbon atoms,

$R_2$ represents a hydrogen, chlorine or bromine atom, a hydroxy, alkoxy, phenylalkoxy or alkyl group, whilst each alkyl moiety may contain from 1 to 3 carbon atoms, or

$R_1$ and $R_2$ together represent an alkylenedioxy group with 1 or 2 carbon atoms,

$R_3$ represents a hydrogen, chlorine or bromine atom or an alkyl group with 1 to 4 carbon atoms,

$R_4$ represents a hydrogen atom, an alkyl or phenylalkyl group, wherein the alkyl moiety may contain from 1 to 3 carbon atoms,

$R_5$ represents a hydrogen, fluorine, chlorine or bromine atom, or an alkyl, hydroxy, alkoxy or phenylalkoxy group wherein each alkyl moiety may contain 1 to 3 carbon atoms,

$R_6$ represents a hydrogen atom or an alkoxy group with 1 to 3 carbon atoms and

$R_7$ represents a hydrogen atom, an alkenyl group with 3 to 5 carbon atoms, an alkyl or phenylalkyl group, wherein the alkyl moiety may contain from 1 to 3 carbon atoms, and the acid addition salts thereof.

2. Indole derivatives of general formula Ia as claimed in claim 1,

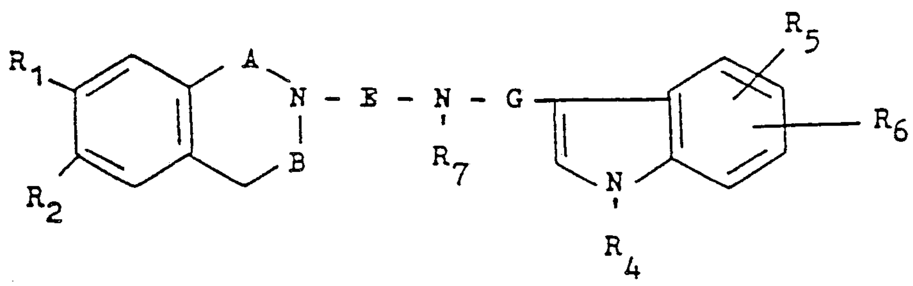

(Ia)

wherein

A and B are defined as in claim 1,

E represents an n-propylene group,

G represents an ethylene or n-propylene group,

$R_1$ represents a chlorine or bromine atom, a methyl, methoxy, nitro, amino, methylamino or dimethylamino group,

$R_2$ represents a hydrogen, chlorine or bromine atom or a methoxy group or $R_1$ and $R_2$ together represent a methylenedioxy group,

$R_4$ represents a hydrogen atom or a methyl group,

$R_5$ represents a hydrogen, fluorine, chlorine or bromine atom or a methyl, hydroxy, methoxy or benzyloxy group,

$R_6$ represents a hydrogen atom or a methoxy group and

$R_7$ represents a hydrogen atom or a methyl group, and the acid addition salts thereof.

3. Indole derivatives of general formula Ia as claimed in claim 2, wherein

A represents a $-CH_2CH_2$ or $-CH=CH$ and B represents a carbonyl group or

A represents a $-COCO$ or $-NH-CO$ group and

B represents a methylene group,

E represents an n-propylene group,

G represents an ethylene group,

$R_1$ and $R_2$ each represent a methoxy group,

$R_4$, $R_5$ and $R_6$ each represent a hydrogen atom and

$R_7$ represents a methyl group, and the acid addition salts thereof.

4. 1-(7,8-Dimethoxy-1,3,4,5-tetrahydro-2H-3-benzazepin-2-on-3-yl)-3-[N-methyl-N-(2-(indolyl-3)-ethyl)amino] propane and the acid addition salts thereof.

5. 1-(7,8-Dimethoxy-1,3,4,5-tetrahydro-2H-3-benzazepin-1,2-dion-3-yl)-3-[N-methyl-N-(2-(indolyl-3)-ethyl)-amino]-propane and the acid addition salts thereof.

6. Physiologically acceptable acid addition salts of the compounds as claimed in claims 1 to 5 with inorganic or organic acids.

7. Pharmaceutical compositions containing a compound of general formula I as claimed in claims 1 to 5 or a physiologically acceptable acid addition salt thereof as claimed in claim 6 in addition to one or more inert carriers and/or diluents.

8. Process for preparing a pharmaceutical composition for the treatment of sinus tachycardia and

ischaemic heart disease, characterised in that, by a non-chemical method, a compound as claimed in claims 1 to 5 or a physiologically acceptable acid addition salt thereof as claimed in claim 6 is incorporated in one or more inert carriers and/or diluents.

9. Use of a compound as claimed in claims 1 to 6 for the preparation of a pharmaceutical composition suitable for the treatment of sinus tachycardia and ischaemic heart disease.

10. Process for preparing new indole derivatives as claimed in claims 1 to 6, characterised in that

a) a compound of general formula II

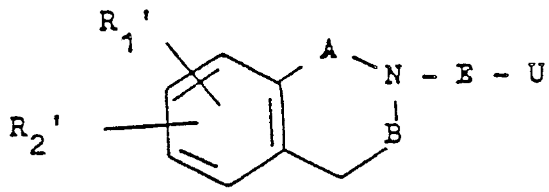

(II)

is reacted with a compound of general formula III

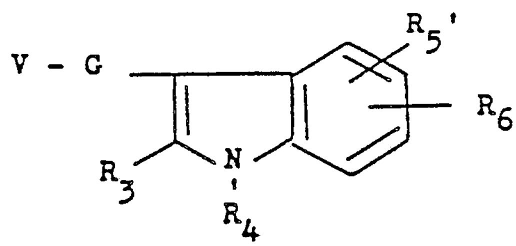

(III)

wherein

$R_3$, $R_4$, $R_6$, A, B, E and G are as hereinbefore defined,

$R_1'$ represents a hydroxy, amino or alkylamino group protected by a protecting group or has the meanings given for $R_1$ hereinbefore,

$R_2'$ represents a hydroxy group protected by a protecting group or has the meanings given for $R_2$ hereinbefore,

$R_5'$ represents a hydroxy group protected by a protecting group or has the meanings given for $R_5$ hereinbefore, one of the groups U or V represents the group $R_7'$—NH, wherein $R_7'$ represents a protecting group for an amino group or has the meanings given for $R_7$ hereinbefore, and the other group U or V represents a nucleophilic leaving group such as a halogen atom or a sulphonyloxy group and subsequently, if desired, any protecting group is split off, or

b) in order to prepare compounds of general formula I wherein B represents a thiocarbonyl group, a compound of general formula IV

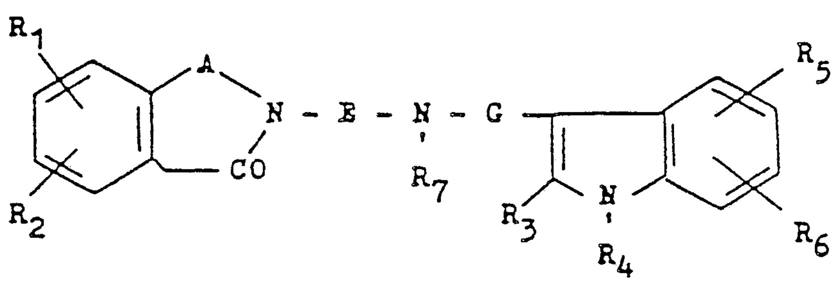

(IV)

wherein $R_1$ to $R_7$, A, E and G are as hereinbefore defined, is reacted with a sulphur-introducing agent, or

c) in order to prepare compounds of general formula I wherein A represents a

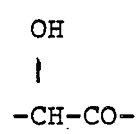

group, a compound of general formula V

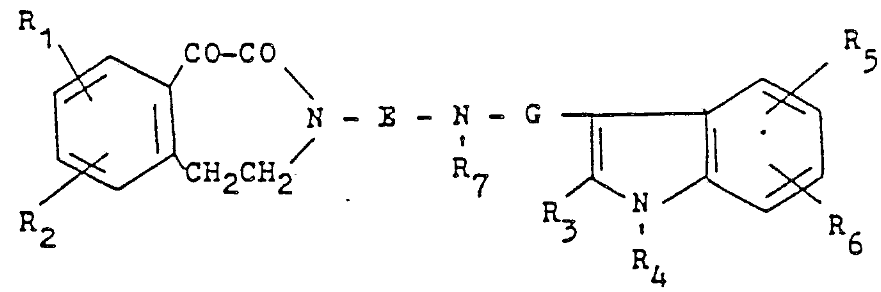

(V)

wherein $R_1$ to $R_7$, E and G are as hereinbefore defined, is reduced, or

d) in order to prepare compounds of general formula I wherein A represents a $-CH_2-CH_2$ or $-CH=CH$ and B represents a methylene group : a compound of general formula VI

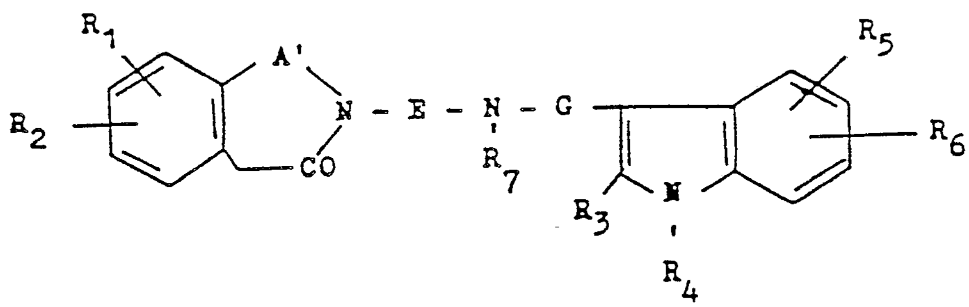

(VI)

wherein

$R_1$ to $R_7$, E and G are as hereinbefore defined and

A' represents a $-CH_2-CH_2$ or $-CH=CH$ group, is reduced, or

e) in order to prepare compounds of general formula I wherein A represents a —COCO group, a compound of general formula VII

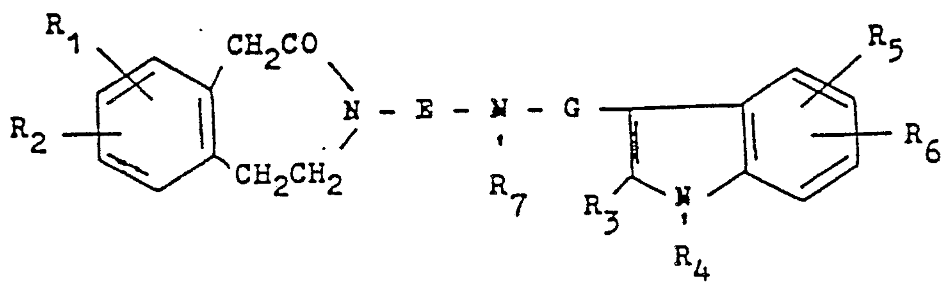

(VII)

wherein $R_1$ to $R_7$, E and G are as hereinbefore defined, is oxidised, or

f) in order to prepare compounds of general formula I wherein A represents a —NH—CO group and E represents an alkylene group with 2 to 4 carbon atoms optionally substituted by an alkyl group with 1 to 3 carbon atoms : a compound of general formula VIII

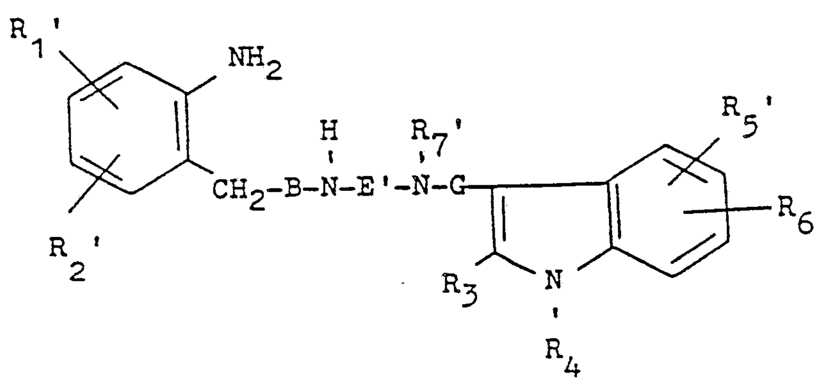

(VIII)

wherein

B, G, $R_3$, $R_4$ and $R_6$ are as hereinbefore defined,

E′ represents an alkenyl group with 2 to 4 carbon atoms optionally substituted by an alkyl group with 1 to 3 carbon atoms,

$R_1'$ represents a hydroxy, amino or alkylamino group protected by a protecting group, or has the meanings given for $R_1$ hereinbefore,

$R_2'$ represents a hydroxy group protected by a protecting group or has the meanings given for $R_2$ hereinbefore,

$R_5'$ represents a hydroxy group protected by a protecting group or has the meanings given for $R_5$ hereinbefore,

$R_7'$ represents a protecting group for an amino group or has the meanings given for $R_7$ hereinbefore, with the exception of hydrogen, is reacted with a carbonic acid derivative of general formula IX

$$W-CO-W \qquad (IX)$$

wherein W, which may be identical or different, each represent a nucleophilic group, and subsequently, if desired, any protecting group used is split off and if desired, subsequently, a compound of general formula I thus obtained is converted into an acid addition salt thereof, particularly a physiologically acceptable acid addition salt with an inorganic or organic acid.

## Revendications

1. Dérivés d'indole de formule générale I

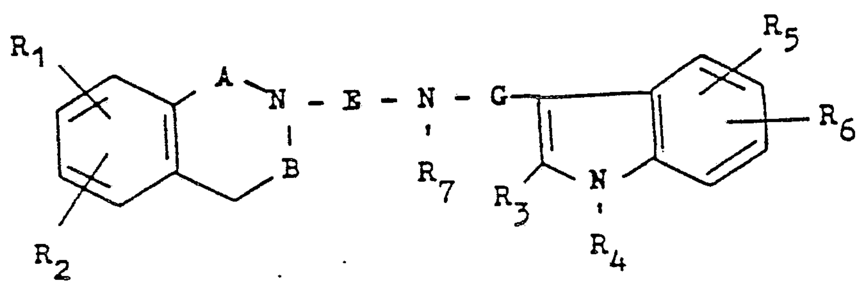

(I)

dans laquelle

A représente un groupe $-CH_2-CH_2-$, $-CH=CH-$, $-\underset{x}{NH}-CO-$ ou $-\underset{x}{CH_2}-CO-$

et

B représente un groupe méthylène, carbonyle ou thiocarbonyle ou

A représente un groupe $-CO-CO-$ ou

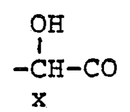

et B représente un groupe méthylène, l'atome de carbone marqué par x étant dans chaque cas relié au noyau phényle,

E représente un groupe alcoylène rectiligne, présentant 2 à 4 atomes de carbone, éventuellement substitué par un groupe alcoyle avec 1 à 3 atomes de carbone, ou représente un groupe 2-hydroxy-n-propylène, 2-hydroxy-n-butylène ou 3-hydroxy-n-butylène,

G représente un groupe alcoylène rectiligne, présentant 1 à 5 atomes de carbone, éventuellement substitué par un groupe alcoyle avec 1 à 3 atomes de carbone,

$R_1$ représente un atome d'hydrogène, de chlore ou de brome, un groupe trifluorométhyle, nitro, amino, alcoylamino, dialcoylamino, alcoyle, hydroxy, alcoxy, ou phénylalcoxy, chaque partie alcoyle pouvant contenir à chaque fois 1 à 3 atomes de carbone,

$R_2$ représente un atome d'hydrogène, de chlore ou de brome, un groupe hydroxy, alcoxy, phénylalcoxy ou alcoyle, chaque partie alcoyle pouvant contenir à chaque fois 1 à 3 atomes de carbone, ou

$R_1$ et $R_2$ représentent ensemble un groupe alcoylènedioxy avec 1 à 2 atomes de carbone,

$R_3$ représente un atome d'hydrogène, de chlore ou de brome ou un groupe alcoyle avec 1 à 4 atomes de carbone,

$R_4$ représente un atome d'hydrogène, un groupe alcoyle ou phénylalcoyle, la partie alcoyle pouvant contenir à chaque fois 1 à 3 atomes de carbone,

$R_5$ représente un atome d'hydrogène, de fluor, de chlore ou de brome, un groupe alcoyle, hydroxy, alcoxy ou phénylalcoxy, chaque partie alcoyle pouvant à chaque fois contenir 1 à 3 atomes de carbone,

$R_6$ représente un atome d'hydrogène ou un groupe alcoxy avec 1 à 3 atomes de carbone, et

$R_7$ représente un atome d'hydrogène, un groupe alcényle avec 3 à 5 atomes de carbone, un groupe alcoyle ou phénylalcoyle, la partie alcoyle pouvant à chaque fois contenir 1 à 3 atomes de carbone, et leurs sels d'addition d'acides.

2. Dérivés d'indole de formule générale Ia selon la revendication 1

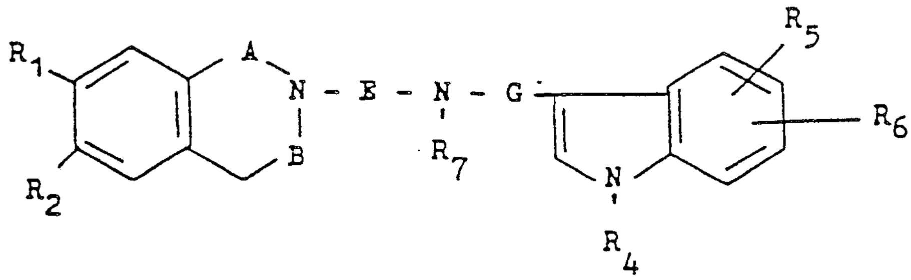

(Ia)

dans laquelle

A et B sont définis comme dans la revendication 1,

E représente un groupe n-propylène,

G représente un groupe éthylène ou n-propylène,

$R_1$ représente un atome de chlore ou de brome, un groupe méthyle, méthoxy, nitro, amino, méthylamino ou diméthylamino,

$R_2$ représente un atome d'hydrogène de chlore ou de brome, un groupe méthoxy, ou $R_1$ et $R_2$ représentent ensemble un groupe méthylènedioxy,

$R_4$ représente un atome d'hydrogène ou un groupe méthyle,

$R_5$ représente un atome d'hydrogène, de fluor, de chlore ou de brome, un groupe méthyle, hydroxy, méthoxy ou benzyloxy,

$R_6$ représente un atome d'hydrogène ou un groupe méthoxy, et

$R_7$ représente un atome d'hydrogène ou un groupe méthyle, et leurs sels d'addition d'acides.

3. Dérivés d'indole de formule générale Ia selon la revendication 2, dans laquelle

A représente un groupe $-CH_2-CH_2-$ ou $-CH=CH-$ et B représente un groupe carbonyle ou

A représente un groupe $-COCO-$ ou $-NH-CO-$ et B représente un groupe méthylène,

E représente un groupe n-propylène,

G représente un groupe éthylène,

$R_1$ et $R_2$ représentent chacun un groupe méthoxy,

$R_4$, $R_5$ et $R_6$ représentent chacun un atome d'hydrogène et

$R_7$ représente un groupe méthyle, et leurs sels d'addition d'acides.

4. Le 1-(7,8-diméthoxy-1,3,4,5-tétrahydro-2H-3-benzazépine-2-one-3-yl)-3-[N-méthyl-N-(2-(indolyl-3)-éthyl)-amino]-propane et ses sels d'addition d'acides.

5. Le 1-(7,8-diméthoxy-1,3,4,5-tétrahydro-2H-3-benzazépine-1,2-dione-3-yl)-3-[N-méthyl-N-(2-(indolyl-3)-éthyl)-amino]-propane et ses sels d'addition d'acides.

6. Sels d'addition d'acides physiologiquement supportables des composés selon les revendications 1 à 5 avec des acides minéraux ou organiques.

7. Médicament contenant un composé de formule générale I selon les revendications 1 à 5 ou son sel d'addition d'acide physiologiquement supportable selon la revendication 6, conjointement à un ou plusieurs excipients et/ou diluants inertes.

8. Procédé pour la fabrication d'un médicament pour le traitement des tachycardies sinusales et des maladies cardiaques ischémiques, caractérisé en ce qu'on introduit, par voie non chimique, un composé selon les revendications 1 à 5 ou son sel d'addition d'acide physiologiquement supportable selon la revendication 6 dans un ou plusieurs excipients et/ou diluants inertes.

9. Utilisation d'un composé selon les revendications 1 à 6 pour la fabrication d'un médicament qui convient au traitement des tachycardies sinusales et des maladies cardiaques ischémiques.

10. Procédé pour la préparation des nouveaux dérivés d'indole selon les revendications 1 à 6, caractérisé en ce que

a) on fait réagir un composé de formule générale II

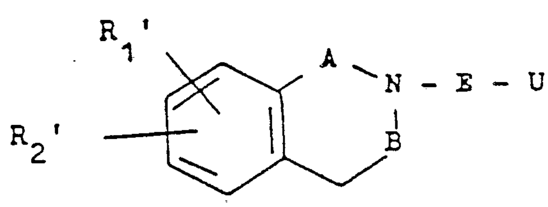

(II)

avec un composé de formule générale III

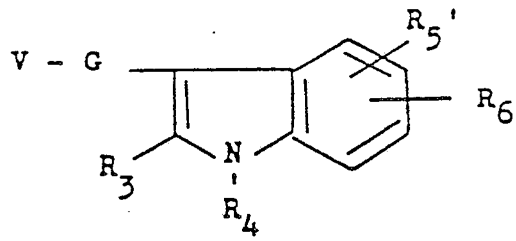

(III)

dans lesquelles

$R_3$, $R_4$, $R_6$, A, B, E et G sont définis comme au début,

$R_1'$ représente un groupe hydroxy, amino ou alcoylamino protégé par un groupe protecteur ou possède les significations mentionnées au début pour $R_1$,

$R_2'$ représente un groupe hydroxy protégé par un groupe protecteur ou possède les significations mentionnées au début pour $R_2$,

$R_5'$ représente un groupe hydroxy protégé par un groupe protecteur ou possède les significations mentionnées au début pour $R_5$, l'un des radicaux U ou V représente le groupe $R_7'$—NH dans lequel $R_7'$ représente un groupe protecteur pour un groupe amino ou possède les significations mentionnées au début pour $R_7$, et l'autre des radicaux U ou V représente un groupe partant nucléophile tel qu'un atome d'halogène ou un groupe sulfonyloxy et en ce qu'éventuellement on clive ensuite un groupe protecteur utilisé ou

b) pour la préparation de composés de formule générale I dans laquelle B représente un groupe thiocarbonyle, on fait réagir un composé de formule générale IV

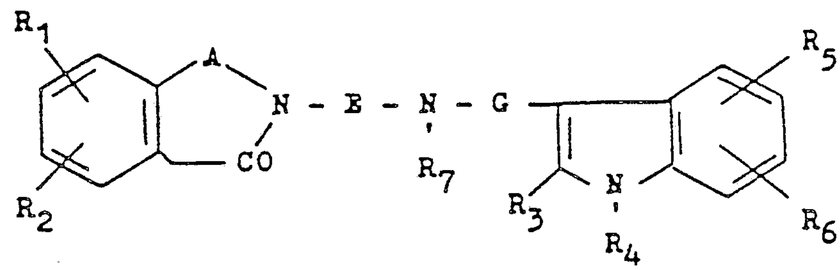

(IV)

dans laquelle $R_1$ à $R_7$, A, E et G sont définis comme au début, avec un agent introduisant du soufre ou

c) pour la préparation de composés de formule générale I dans laquelle A représente un groupe

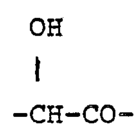

on réduit un composé de formule générale V

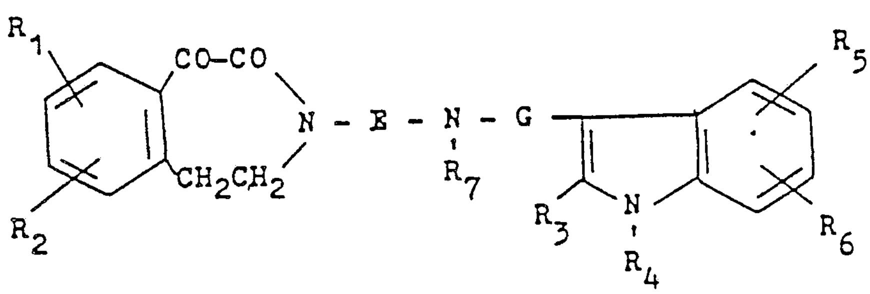

(V)

dans laquelle $R_1$ à $R_7$, E et G sont définis comme au début ou

d) pour la préparation de composés de formule générale I dans laquelle A représente un groupe $—CH_2—CH_2—$ ou $—CH=CH—$ et B un groupe méthylène, on réduit un composé de formule générale VI

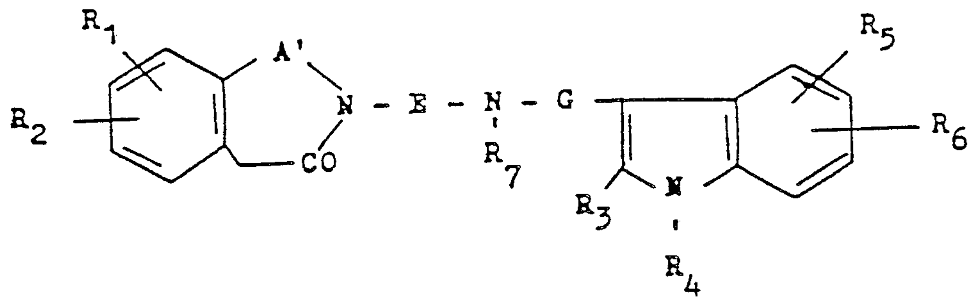

(VI)

dans laquelle

$R_1$ à $R_7$, E et G sont définis comme au début et

A' représente un groupe $-CH_2-CH_2-$ ou $-CH=CH-$, ou

e) pour la préparation de composés de formule générale I dans laquelle A représente le groupe —COCO—, on oxyde un composé de formule générale VII

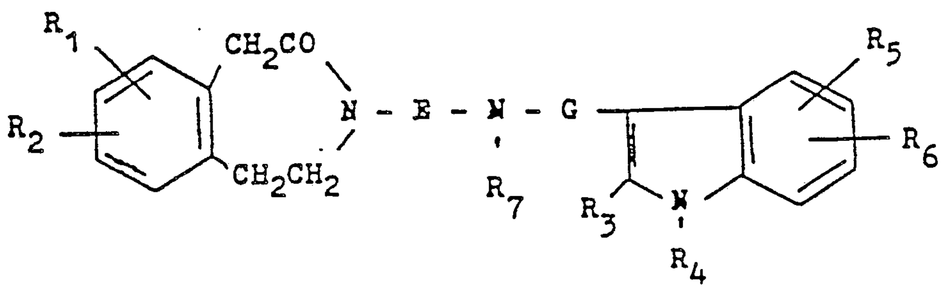

(VII)

dans laquelle $R_1$ à $R_7$, E et G sont définis au début, ou

f) pour la préparation de composés de formule générale I dans laquelle A représente le groupe —NH—CO— et E représente un groupe alcoylène avec 2 à 4 atomes de carbone, éventuellement substitué par un groupe alcoyle avec 1 à 3 atomes de carbone, on fait réagir un composé de formule générale VIII

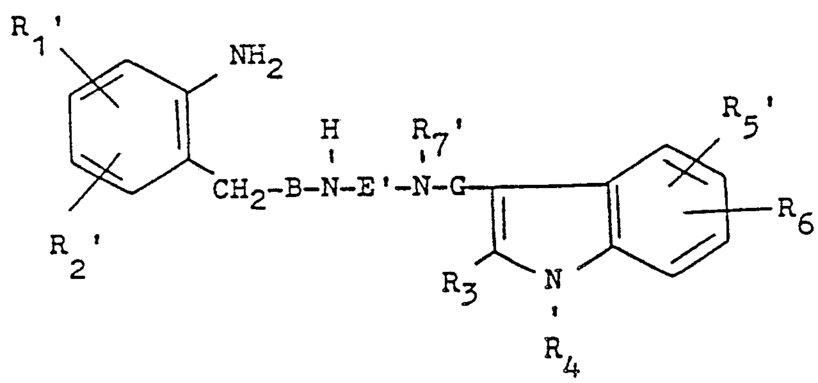

(VIII)

dans laquelle

B, G, $R_3$, $R_4$ et $R_6$ sont définis comme au début,

E' représente un groupe alcényle avec 2 à 4 atomes de carbone, éventuellement substitué par un groupe alcoyle avec 1 à 3 atomes de carbone,

$R_1'$ représente un groupe hydroxy, amino ou alcoylamino protégé par un groupe protecteur ou possède les significations mentionnées au début pour $R_1$,

$R_2'$ représente un groupe hydroxy protégé par un groupe protecteur ou possède les significations mentionnées au début pour $R_2$,

$R_5'$ représente un groupe hydroxy protégé par un groupe protecteur ou possède les significations mentionnées au début pour $R_5$,

$R_7'$ représente un groupe protecteur pour un groupe amino ou possède les significations mentionnées au début pour $R_7$, à l'exception de l'hydrogène, avec un dérivé d'acide carbonique de formule générale IX

$$W-CO-W \qquad \text{(IX)}$$

dans laquelle W, qui peuvent être identiques ou différents, représentent chacun un groupe partant nucléophile et on clive éventuellement ensuite un groupe protecteur utilisé et si on le désire, on transforme ensuite un composé ainsi obtenu de formule générale I en son sel d'addition d'acide, en particulier en son sel d'addition d'acide physiologiquement supportable, avec un acide minéral ou organique.